# EUROPEAN PATENT APPLICATION

(11) **EP 3 456 353 A1**
(43) Date of publication of application: **20.03.2019**
(21) Application number: 17796144.8
(22) Date of filing: 09.05.2017
(51) Int. Cl.: A61K 45/00, A61K 31/14, A61K 31/439, A61K 31/4425, A61K 31/46, A61K 31/4725, A61K 31/485, A61K 31/5513, A61K 38/46, A61K 39/395, A61K 48/00, A61P 17/00, A61P 37/08, A61P 43/00

(54) **COMPOSITION FOR TREATING OR PREVENTING ATOPIC DERMATITIS**

(30) Priority: 09.05.2016 JP 2016093918
(71) Applicant: Nanoegg Research Laboratories, Inc., Minato-ku, Tokyo 107-0052 (JP)
(72) Inventor: YAMAGUCHI Yoko, Kawasaki-shi Kanagawa 210-0821 (JP); NAGASAWA Teruaki, Kawasaki-shi Kanagawa 210-0821 (JP); KUBOTA Yoshiki, Kawasaki-shi Kanagawa 210-0821 (JP); SHIMURA Nanako, Kawasaki-shi Kanagawa 210-0821 (JP); MUSASHI Mina, Kawasaki-shi Kanagawa 210-0821 (JP)
(74) Representative: Uexküll & Stolberg
(86) International application number: PCT/JP2017/017565
(87) International publication number: WO 2017/195783

(57) **Abstract**

The present invention provides a composition and a method both for treating or preventing atopic dermatitis effectively. Provided is a composition which contains an acetylcholine inhibitor, e.g., an anticholinergic substance, as an active ingredient. Also provided is a method which includes a step of administering an acetylcholine inhibitor such as an anticholinergic substance. The detailed mechanism of the development of atopic dermatitis has not been elucidated so far. Therefore, it has been difficult to treat atopic dermatitis effectively. The composition according to the present invention can treat atopic dermatitis effectively through the inhibition of the action of acetylcholine.

## Description

### [Technical Field]

The present invention relates to a composition for treating or preventing atopic dermatitis.

### [Background Art]

Atopic dermatitis is dermatitis with prurigo, which is strongly associated with an allergic reaction to food (egg white, milk, soy bean, or the like) or an antigen (mite, dust, pollen, mold, or the like). Atopic dermatitis may develop within several months after birth and become recurrent, severe, or chronic at 10 years old or older.

Therapeutic agents for atopic dermatitis include steroidal topical agents as the first option. Meanwhile, steroidal agents can only suppress inflammation due to atopic dermatitis. Steroidal agents cannot directly treat atopic dermatitis. Steroidal agents can also result in side effects such as induction of a skin infection due to suppression of the immune system of the skin surface.

The stratum corneum of atopic dermatitis patients is known to have a reduced amount of ceramide in the intercellular lipid, resulting in feeling of dryness on the skin. It has been reported that systemic application of a moisturizing agent immediately after birth to prevent dryness of the skin has reduced atopic dermatitis by 30% (Non Patent Literature 1). A deletion mutation of filaggrin has also been reported as the cause of developing atopic dermatitis (Non Patent Literature 2). Meanwhile, the detailed mechanism of developing atopic dermatitis has not been elucidated so that an effective therapy had not been established.

### [Citation List]

### [Non Patent Literature]

[NPL 1] Horimukai K, et al., J Allergy Clin Immunol. 2014 Oct; 134(4): 824-830.
[NPL 2] Sandilands A et al., J Cell Sci. 2009 May 1; 122 (Pt 9): 1285-94

### [Summary of Invention]

### [Technical Problem]

Since the detailed mechanism of developing atopic dermatitis had not been elucidated as discussed above, an effective therapeutic target for atopic dermatitis could not be found. Therefore, atopic dermatitis could not be effectively treated. The objective of the present invention is to provide a novel therapeutic drug for effectively treating atopic dermatitis.

### [Solution to Problem]

The inventors have completed the present invention as a result of diligent study by newly discovering that elevation in the acetylcholine concentration is deeply involved as the cause of developing atopic dermatitis, and providing an inhibitor of acetylcholine as the active ingredient for preventing/treating atopic dermatitis.

The present invention provides, for example, the following items.

### (Item 1)

A composition for treating or preventing atopic dermatitis, comprising an inhibitor of acetylcholine.

### (Item 2)

The composition of item 1, wherein the inhibitor of acetylcholine is selected from the group consisting of an anti-choline agent, acetylcholinesterase, a nucleic acid encoding acetylcholinesterase, an antibody blocking the binding of acetylcholine to an acetylcholine receptor, a nucleic acid encoding an antibody blocking the binding of acetylcholine to an acetylcholine receptor, an antibody to an acetylcholine receptor, a nucleic acid encoding an antibody to an acetylcholine receptor, an agent inhibiting the gene expression of an acetylcholine receptor, and an inhibitor of choline acetyltransferase.

### (Item 3)

The composition of item 1, wherein the inhibitor of acetylcholine is an anti-choline agent.

### (Item 4)

The composition of item 1, which is free of crystal violet.

### (Item 5)

The composition of item 3, wherein the anti-choline agent is an antagonist of an acetylcholine receptor.

### (Item 6)

The composition of item 5, wherein the antagonist of an acetylcholine receptor is selected from the group consisting of trihexyphenidyl hydrochloride, scopolamine butylbromide, pirenzepine hydrochloride hydrate, ipratropium bromide, oxitropium bromide, tiotropium bromide, atropine sulfate, tropicamide, diphenhydramine hydrochloride, dicycloverine, oxybutynin hydrochloride, cyclopentrate hydrochloride, tolterodine tartrate, solifenacin succinate, darifenacin hydrobromide, mevevelin hydrochloride, procyclidine hydrochloride, acridinium bromide, propantheline bromide, scopolamine hydrobromide hydrate, metoscopolamine bromide, mebenzolate bromide, methanethelinium bromide, orphenadrine citrate, homatropine hydrobromide, prefinium bromide, methixene hydrochloride, pipethanate ethobromide, adiphenin hydrochloride, mazaticol hydrochloride hydrate, eutropin chloride, imidafenacin, fesoterodine fumarate, suxamethonium chloride hydrate, decamethonium bromide, vecuronium bromide, pancuronium bromide, d-tubocurarine chloride hydrochloride hydrate, gallamintriethiodide, mecamylamine hydrochloride, trimetaphan camsilate, hexamethonium bromide, atracurium besilate, doxacurium chloride, mivacurium chloride, 18-methoxycoronaridine, dextromethorphan hydrobromide hydrate, methyllycaconitine, α-bungarotoxin, α-conotoxin G1, benzoquinonium, and bPiDDB, and analogs, mimetics, and derivatives of these substances with anti-choline action.

### (Item 7)

The composition of item 3, wherein the anti-choline agent is a partial agonist or an inverse agonist of an acetylcholine receptor.

### (Item 8)

The composition of item 7, wherein the anti-choline agent is a partial agonist of an acetylcholine receptors selected from group consisting of TAB, Hexyl-TAB, sabcomeline, and milameline, and analogs, mimetics, and derivatives of these substances with anti-choline action.

### (Item 9)

The composition of item 7, wherein the anti-choline agent is an inverse agonist of an acetylcholine receptor selected from the group consisting of pirenzepine and analogs, mimetics, and derivatives of these substances with anti-choline action.

### (Item 10)

The composition of item 3, wherein the anti-choline agent is a compound having a structure comprising one or more tertiary amino groups and an alkyl chain with 1 to 9 carbons binding thereto.

### (Item 11)

The composition of item 10, wherein the number of carbons of the alkyl chain is 1 to 6.

### (Item 12)

The composition of item 10, wherein three substituents on nitrogen of the tertiary amino group are methyl groups.

### (Item 13)

The composition of item 1, wherein the inhibitor of acetylcholine is acetylcholinesterase.

### (Item 14)

The composition of item 1, wherein the inhibitor of acetylcholine is an antibody blocking the binding of acetylcholine to an acetylcholine receptor.

### (Item 15)

The composition of item 1, wherein the inhibitor of acetylcholine is an agent inhibiting the gene expression of an acetylcholine receptor.

### (Item 16)

The composition of item 1, wherein the inhibitor of acetylcholine is an inhibitor of choline acetyltransferase.

The present invention further provides, for example, the following items.

### (Item A1)

A method of treating or preventing atopic dermatitis in a subject, comprising administering an inhibitor of acetylcholine to the subject.

### (Item A2)

The method of item A1, wherein the inhibitor of acetylcholine is selected from the group consisting of an anti-choline agent, acetylcholinesterase, a nucleic acid encoding acetylcholinesterase, an antibody blocking the binding of acetylcholine to an acetylcholine receptor, a nucleic acid encoding an antibody blocking the binding of acetylcholine to an acetylcholine receptor, an antibody to an acetylcholine receptor, a nucleic acid encoding an antibody to an acetylcholine receptor, an agent inhibiting the gene expression of an acetylcholine receptor, and an inhibitor of choline acetyltransferase.

### (Item A3)

The method of item A1, wherein the inhibitor of acetylcholine is an anti-choline agent.

### (Item A4)

The method of item A1, wherein the inhibitor of acetylcholine is free of crystal violet.

### (Item A5)

The method of item A3, wherein the anti-choline agent is an antagonist of an acetylcholine receptor.

### (Item A6)

The method of item A5, wherein the antagonist of the acetylcholine receptor is selected from the group consisting of trihexyphenidyl hydrochloride, scopolamine butylbromide, pirenzepine hydrochloride hydrate, ipratropium bromide, oxitropium bromide, tiotropium bromide, atropine sulfate, tropicamide, diphenhydramine hydrochloride, dicycloverine, oxybutynin hydrochloride, cyclopentrate hydrochloride, tolterodine tartrate, solifenacin succinate, darifenacin hydrobromide, mevevelin hydrochloride, procyclidine hydrochloride, acridinium bromide, propantheline bromide, scopolamine hydrobromide hydrate, metoscopolamine bromide, mebenzolate bromide, methanethelinium bromide, orphenadrine citrate, homatropine hydrobromide, prefinium bromide, methixene hydrochloride, pipethanate ethobromide, adiphenin hydrochloride, mazaticol hydrochloride hydrate, eutropin chloride, imidafenacin, fesoterodine fumarate, suxamethonium chloride hydrate, decamethonium bromide, vecuronium bromide, pancuronium bromide, d-tubocurarine chloride hydrochloride hydrate, gallamintriethiodide, mecamylamine hydrochloride, trimetaphan camsilate, hexamethonium bromide, atracurium besilate, doxacurium chloride, mivacurium chloride, 18-methoxycoronaridine, dextromethorphan hydrobromide hydrate, methyllycaconitine, α-bungarotoxin, α-conotoxin G1, benzoquinonium, and bPiDDB, and analogs, mimetics, and derivatives of these substances with anti-choline action.

### (Item A7)

The method of item A3, wherein the anti-choline agent is a partial agonist or an inverse agonist of an acetylcholine receptor.

### (Item A8)

The method of item A7, wherein the anti-choline agent is a partial agonist of an acetylcholine receptor selected from group consisting of TAB, Hexyl-TAB, sabcomeline, and milameline, and analogs, mimetics, and derivatives of these substances with anti-choline action.

### (Item A9)

The method of item A7, wherein the anti-choline agent is an inverse agonist of an acetylcholine receptor selected from the group consisting of pirenzepine and analogs, mimetics, and derivatives of these substances with anti-choline action.

### (Item A10)

The method of item A3, wherein the anti-choline agent is a compound having a structure comprising one or more tertiary amino groups and an alkyl chain with 1 to 9 carbons binding thereto.

### (Item A11)

The method of item A10, wherein the number of carbons of the alkyl chain is 1 to 6.

### (Item A12)

The method of item A10, wherein three substituents on nitrogen of the tertiary amino group are methyl groups.

### (Item A13)

The method of item A1, wherein the inhibitor of acetylcholine is acetylcholinesterase.

### (Item A14)

The method of item A1, wherein the inhibitor of acetylcholine is an antibody blocking the binding of acetylcholine to an acetylcholine receptor.

### (Item A15)

The method of item A1, wherein the inhibitor of acetylcholine is an agent inhibiting the gene expression of an acetylcholine receptor.

### (Item A16)

The method of item A1, wherein the inhibitor of acetylcholine is an inhibitor of choline acetyltransferase.

The present invention further provides, for example, the following items.

### (Item B1)

A composition or combination for treating or preventing atopic dermatitis, comprising an inhibitor of muscarinic acetylcholine and an inhibitor of nicotinic acetylcholine.

### (Item B2)

The composition or combination of item B1, wherein the inhibitor of muscarinic acetylcholine is an antagonist of a muscarinic acetylcholine receptor, and the inhibitor of nicotinic acetylcholine is an antagonist of a nicotinic acetylcholine receptor.

### (Item B3)

The composition or combination of item B2,
wherein the antagonist of a muscarinic acetylcholine receptor is selected from the group consisting of trihexyphenidyl hydrochloride, scopolamine butylbromide, pirenzepine hydrochloride hydrate, ipratropium bromide, oxitropium bromide, tiotropium bromide, atropine sulfate, tropicamide, diphenhydramine hydrochloride, dicycloverine, oxybutynin hydrochloride, cyclopentrate hydrochloride, tolterodine tartrate, solifenacin succinate, darifenacin hydrobromide, mevevelin hydrochloride, procyclidine hydrochloride, acridinium bromide, propantheline bromide, scopolamine hydrobromide hydrate, metoscopolamine bromide, mebenzolate bromide, methanethelinium bromide, orphenadrine citrate, homatropine hydrobromide, prefinium bromide, methixene hydrochloride, pipethanate ethobromide, adiphenin hydrochloride, mazaticol hydrochloride hydrate, eutropin chloride, imidafenacin, and fesoterodine fumarate, and
wherein the antagonist of a nicotinic acetylcholine receptor is selected from the group consisting of suxamethonium chloride hydrate, decamethonium bromide, vecuronium bromide, pancuronium bromide, d-tubocurarine chloride hydrochloride hydrate, gallamintriethiodide, mecamylamine hydrochloride, trimetaphan camsilate, hexamethonium bromide, atracurium besilate, doxacurium chloride, mivacurium chloride, 18-methoxycoronaridine, dextromethorphan hydrobromide hydrate, methyllycaconitine, α-bungarotoxin, α-conotoxin G1, benzoquinonium, and bPiDDB.

### (Item B4)

The composition or combination of item B3,
wherein the antagonist of a muscarinic acetylcholine receptor is selected from the group consisting of solifenacin succinate, acridinium bromide, and scopolamine hydrobromide hydrate, and
wherein the antagonist of a nicotinic acetylcholine receptor is selected from the group consisting of d-tubocurarine chloride hydrochloride hydrate, gallamintriethiodide, hexamethonium bromide, dextromethorphan hydrobromide hydrate, and α-bungarotoxin.

### (Item B5)

The composition or combination of item B4, wherein the antagonist of a muscarinic acetylcholine receptor is scopolamine hydrobromide hydrate, and the antagonist of a nicotinic acetylcholine receptor is hexamethonium bromide.

### (Item B6)

The composition or combination of any one of items 1 to 16 and B1 to B5, characterized in that the composition or combination is administered by topical application, transdermal administration, intradermal administration, subcutaneous administration, intravenous administration, oral administration, enteral administration, pulmonary administration, transnasal administration, intraperitoneal administration, ear drops, or eye drops.

### (Item B7)

The combination of any one of items B1 to B6, characterized in that the inhibitor of muscarinic acetylcholine and the inhibitor of nicotinic acetylcholine are simultaneously or successively administered.

### (Item C1)

A method of treating or preventing atopic dermatitis in a subject, comprising administering an inhibitor of muscarinic acetylcholine and an inhibitor of nicotinic acetylcholine to the subject.

### (Item C2)

The method of item C1, wherein the inhibitor of muscarinic acetylcholine is an antagonist of a muscarinic acetylcholine receptor, and the inhibitor of nicotinic acetylcholine is an antagonist of a nicotinic acetylcholine receptor.

### (Item C3)

The method of item C2,
wherein the antagonist of a muscarinic acetylcholine receptor is selected from the group consisting of trihexyphenidyl hydrochloride, scopolamine butylbromide, pirenzepine hydrochloride hydrate, ipratropium bromide, oxitropium bromide, tiotropium bromide, atropine sulfate, tropicamide, diphenhydramine hydrochloride, dicycloverine, oxybutynin hydrochloride, cyclopentrate hydrochloride, tolterodine tartrate, solifenacin succinate, darifenacin hydrobromide, mevevelin hydrochloride, procyclidine hydrochloride, acridinium bromide, propantheline bromide, scopolamine hydrobromide hydrate, metoscopolamine bromide, mebenzolate bromide, methanethelinium bromide, orphenadrine citrate, homatropine hydrobromide, prefinium bromide, methixene hydrochloride, pipethanate ethobromide, adiphenin hydrochloride, mazaticol hydrochloride hydrate, eutropin chloride, imidafenacin, and fesoterodine fumarate, and
wherein the antagonist of a nicotinic acetylcholine receptor is selected from the group consisting of suxamethonium chloride hydrate, decamethonium bromide, vecuronium bromide, pancuronium bromide, d-tubocurarine chloride hydrochloride hydrate, gallamintriethiodide, mecamylamine hydrochloride, trimetaphan camsilate, hexamethonium bromide, atracurium besilate, doxacurium chloride, mivacurium chloride, 18-methoxycoronaridine, dextromethorphan hydrobromide hydrate, methyllycaconitine, α-bungarotoxin, α-conotoxin G1, benzoquinonium, and bPiDDB.

### (Item C4)

The method of item C3,
wherein the antagonist of a muscarinic acetylcholine receptor is selected from the group consisting of solifenacin succinate, acridinium bromide, and scopolamine hydrobromide hydrate, and
wherein the antagonist of a nicotinic acetylcholine receptor is selected from the group consisting of d-tubocurarine chloride hydrochloride hydrate, gallamintriethiodide, hexamethonium bromide, dextromethorphan hydrobromide hydrate, and α-bungarotoxin.

### (Item C5)

The method of item C4, wherein the antagonist of a muscarinic acetylcholine receptor is scopolamine hydrobromide hydrate, and the antagonist of a nicotinic acetylcholine receptor is hexamethonium bromide.

### (Item C6)

The method of any one of items C1 to C5, characterized in that the inhibitor of muscarinic acetylcholine and the inhibitor of nicotinic acetylcholine are administered by topical application, transdermal administration, intradermal administration, subcutaneous administration, intravenous administration, oral administration, enteral administration, pulmonary administration, transnasal administration, intraperitoneal administration, ear drops, or eye drops.

### (Item C7)

The method of any one of items C1 to C6, characterized in that the inhibitor of muscarinic acetylcholine and the inhibitor of nicotinic acetylcholine are simultaneously or successively administered.

The present invention is intended so that one or more of the above features can be provided not only as the explicitly disclosed combinations, but also as other combinations thereof. Additional embodiments and advantages of the present invention are recognized by those skilled in the art by reading and understanding the following detailed explanation as needed.

### [Advantageous Effects of Invention]

The composition and/or method of the present invention can treat atopic dermatitis, which had been difficult to effectively treat, by providing an inhibitor of an acetylcholine receptor as the active ingredient.

### [Brief Description of Drawings]

Figure **1A** shows pictures of the back of the mouse group before application (pre; top row), day 3 from starting application (3d; second row from the top), and day 7 from starting application (7d; third row from the top) of a test formulation (1. acridinium bromide, 2. scopolamine hydrobromide trihydrate, or 3. hexamethonium bromide). The AD score is shown at the bottom left corner of each picture.
Figure **1B** shows pictures of the back of the mouse group before application (pre), day 3 from starting application (3d), and day 7 from starting application (7d) of a test formulation (4. dextromethorphan hydrobromide monohydrate) or 5. saline. The AD score is shown at the bottom left corner of each picture.
Figure 2 shows a graph showing the body weight before application (pre) and on day 7 from starting application (7 days).
Figure 3 shows graphs that summarize the results of AD scores and ΔAD scores (difference in AD scores before application and after starting application). The bars of each group indicate, from the left, before application (pre), day 3 from starting application (3 days), and day 7 from starting application (7 days).
Figure 4 depicts the change in AD scores of each mouse group applied with each test formulation. The horizontal axis indicates the number of days.
Figure **5A** shows microscope images of skin tissue stained with toluidine blue in an atopic dermatitis induced mouse group applied with a test formulation (1. solifenacin succinate, 2. acridinium bromide, 3. scopolamine hydrobromide trihydrate, or 4. tubocurarine chloride pentahydrate).
Figure **5B** shows microscope images of skin tissue stained with toluidine blue in an atopic dermatitis induced mouse group applied with a test formulation (5. gallamintriethiodide, 6. hexamethonium bromide, 7. dextromethorphan hydrobromide monohydrate, or 8. α-bungarotoxin).
Figure 5C shows microscope images of skin tissue stained with toluidine blue in atopic dermatitis induced mouse group applied with saline and mouse group with no atopic dermatitis induction (non-treatment group).
Figure **5D** shows a graph showing the mast cell count at a severe area in an atopic dermatitis induced mouse group applied with a test formulation or saline. The vertical axis indicates the cell count per 1 mm² of skin.
Figure **6A** shows pictures of the back of the mouse group before application (pre; top row), day 3 from starting application (3d; second row from the top), day 7 from starting application (7d; third row from the top), and day 11 from starting application (11d; fourth row from the top) of a test formulation (1. acridinium bromide, 2. scopolamine hydrobromide trihydrate, or 3. hexamethonium bromide). The AD score is shown at the bottom left corner of each picture.
Figure **6B** shows pictures of the back of the mouse group before application (pre), day 3 from starting application (3d), day 7 from starting application (7d), and day 11 from starting application (11d) of a test formulation (4. dextromethorphan hydrobromide monohydrate) or 5. saline. The AD score is shown at the bottom left corner of each picture.
Figure **7** shows a graph showing the body weight before application (pre), on day 3 from starting application (3 days), on day 7 from starting application (7 days), and on day 7 from starting application (7 days) when using each test formulation.
Figure **8** shows a graph that summarizes the results of AD scores and ΔAD scores (difference in AD scores before application and after starting application) upon use of each test formulation. The bars of each group indicate, from the left, before application (pre), day 3 from starting application (3 days), day 7 from starting application (7 days), and day 11 from starting application (11 days).
Figure **9** depicts the change in AD scores of each mouse group applied with each test formulation. The horizontal axis indicates the number of days.
Figure **10** shows comparison of the acetylcholine concentrations in the skin of an atopic dermatitis induced mouse group applied with saline and a mouse group without induction of atopic dermatitis. The vertical axis indicates the amount of acetylcholine (µmol) per kg of skin.
Figure **11** shows the atopic dermatitis score (mean change) in mice (NC/Nga) administered with a test formulation (suxamethonium chloride, TAB, Hexyl-TAB, Cetyl-TAB, hexamethonium bromide, TMHDA, HMDA, or decamethonium bromide) and mite antigen. The vertical axis indicates the atopic dermatitis score (mean change), and the horizontal axis indicates the number of days. As controls, a non-treatment group (no induction and no application of a test formulation) and a solvent (with induction and application of purified water, DW) applied group were used.
Figure 12 shows a comparison of atopic dermatitis scores between an antagonist and agonist. The vertical axis indicates the atopic dermatitis score (mean change), and the horizontal axis indicates the number of days.
Figure **13** shows a comparison of atopic dermatitis scores for different amino group classes. The vertical axis indicates the atopic dermatitis score (mean change), and the horizontal axis indicates the number of days.
Figure **14** shows a comparison of atopic dermatitis scores for different amino group numbers in the molecule. The vertical axis indicates the atopic dermatitis score (mean change), and the horizontal axis indicates the number of days.
Figure **15** shows a comparison of atopic dermatitis scores for different carbon chain lengths. The vertical axis indicates the atopic dermatitis score (mean change), and the horizontal axis indicates the number of days.
Figure **16** shows images of replicas of skin before (pre) and after (post) application of hexamethonium (12 mM) in atopic dermatitis patients.
Figure 17 shows graphs (left panel) showing the mean change in dermatitis scores of a mouse group applied with purified water (DW), a combination of a low concentration of hexamethonium and scopolamine (Hexa. & Scop. Lo.), and a combination of a high concentration of hexamethonium and scopolamine (Hexa. & Scop. Hi.), and shows pictures of the back of mice on day 0 (pre) and day 10 from starting application.
Figure **18** shows a graph (left panel) showing the mean change in dermatitis scores of a mouse group orally administered with hexamethonium, and shows pictures of the back of mice on day 0 (pre) and day 10 from starting application. DW applied mice were used as controls.

### [Description of Embodiments]

The terms used herein should be understood as being used in the meaning that is commonly used in the art, unless specifically noted otherwise. Thus, unless defined otherwise, all terminologies and scientific technical terms that are used herein have the same meaning as the general understanding of those skilled in the art to which the present invention pertains. In case of a contradiction, the present specification (including the definitions) takes precedence.

### (Definitions of terms)

As used herein, "atopic dermatitis" is dermatitis with prurigo, characterized by a chronic or repetitive course of repeating remission and exacerbation. Many instances develop with a family background of allergic asthma, allergic rhinitis (hay fever), allergic conjunctivitis or the like and/or atopic predisposition, which is a predisposition for producing IgE antibodies.

As used herein, "acetylcholine" refers to a neurotransmitter represented by the formula CH₃COO(CH₂)₂N⁺(CH₃)₃, which is released from the parasympathetic nerve or the motor nerve ending. Acetylcholine is abbreviated as ACh. Acetylcholine is distributed throughout the entire neuron, but the concentration is the highest at the nerve endings. Acetylcholinesterase (AChE) decomposes acetylcholine released out of cells into choline and acetic acid. For this reason, the effect of acetylcholine released out of cells is quickly eliminated. The decomposed choline is reincorporated into the cells and reused for the production of acetylcholine.

As used herein, "inhibitor of acetylcholine" refers to a molecule that directly or indirectly inhibits the function of acetylcholine. Thus, examples of inhibitors of acetylcholine include, but are not limited to, compounds inhibiting acetylcholine, anti-choline agents, acetylcholinesterase (or nucleic acids encoding the same), antibodies to an acetylcholine receptor (or nucleic acids encoding the same), agents inhibiting the gene expression of an acetylcholine receptor (e.g., ribozymes, antisense nucleic acids, and siRNA to a gene encoding an acetylcholine receptor), inhibitors of choline acetyltransferase typically, agents inhibiting the gene expression of choline acetyltransferase (e.g., ribozymes, antisense nucleic acids, and siRNA to a gene) and the like.

As used herein, "anti-choline agent" refers to a substance that partially or completely inhibits the action of a neurotransmitter acetylcholine by acting on an acetylcholine receptor. Examples of anti-choline agent include antagonists of an acetylcholine receptor. Agonists of an acetylcholine receptor that ultimately acts towards suppression (e.g., partial agonists and inverse agonists) can also be used an anti-choline agent.

As used herein, "acetylcholinesterase" refers to an enzyme serving the role of decomposing acetylcholine released as a neurotransmitter into choline and acetic acid to eliminate the action of acetylcholine. With such an enzymatic activity, acetylcholinesterase can exert a function as an inhibitor of acetylcholine. Acetylcholinesterase is abbreviated as AChE.

As used herein, "antibody blocking the binding of acetylcholine to an acetylcholine receptor" refers to an antibody that can block the binding of acetylcholine to an acetylcholine receptor by specifically binding to a specific moiety (or the vicinity thereof) of an acetylcholine receptor associated with binding with acetylcholine. Such an antibody can inhibit the action of acetylcholine. Alternatively, an "antibody blocking the binding of acetylcholine to an acetylcholine receptor" can be an acetylcholine binding antibody that inhibits the binding of acetylcholine and an acetylcholine receptor.

As used herein, "siRNA" is an RNA molecule with a double stranded RNA moiety consisting of 15 to 40 bases, which has a function of cleaving the mRNA of a target gene having a sequence that is complementary to the antisense strand of the siRNA to suppress the expression of the target gene. More specifically, the siRNA in the present invention is an RNA comprising a double stranded RNA moiety consisting of a sense RNA strand consisting of a sequence homologous to continuous RNA sequence in mRNA encoding an acetylcholine receptor or the like and an antisense RNA strand consisting of a sequence that is complementary to the sense RNA sequence.

As used herein, "acetylcholine receptor" refers to a protein that specifically recognizes and binds acetylcholine. This is abbreviated as AChR and is also called a cholinergic receptor. Such a receptor with nicotine as an agonist is referred to as a nicotinic acetylcholine receptor, and such a receptor with muscarine as an agonist is referred to as a muscarinic acetylcholine receptor. A "nicotinic acetylcholine receptor" is also referred to as a "nicotine receptor" and abbreviated as nAChR. Two types of nicotinic acetylcholine receptors are known, i.e., nicotinic acetylcholine receptors of the skeletal muscle and nicotinic acetylcholine receptors of the nerve. Skeletal muscle nicotinic acetylcholine receptors are present at the neuromuscular junction at the motor nerve ending, while nicotinic acetylcholine receptors of the nerve are present at the preganglionic fiber endings (ganglion part) of the sympathetic nerve and parasympathetic nerve. A "muscarinic acetylcholine receptor" is also referred to as a "muscarine receptor" and abbreviated as mAChR. Existence of five subtypes of muscarinic acetylcholine receptors from M1 to M5 has been confirmed. The distribution of the five subtypes varies by the organ.

As used herein, "antagonist" refers to a substance that inhibits an effect of a ligand (e.g., neurotransmitter such as acetylcholine, hormone, or the like) by acting on a receptor. An antagonist is also called a blocking agent or a blocker. Antagonists include competitive antagonists that bind to a receptor to obstruct the binding of a ligand to inhibit the action thereof and non-competitive antagonists that act on a site other than a receptor to induce an opposite action from a specific ligand to inhibit the action of the ligand.

As used herein, "agonist" refers to a substance exhibiting a function that is the same or different from the effect of a ligand (e.g., neurotransmitter such as acetylcholine, hormone, or the like) by acting on a receptor. Agonists include agonist that exhibit physiological actions similar to the original action of a ligand and partial agonists that has low action relative to the original ligand even when applied at a high concentration. There are also inverse agonists that exhibit the opposite action from the physiological action of the original ligand by binding to a receptor.

As used herein, "choline acetyltransferase" refers to an enzyme, which catalyzes a reaction of generating acetylcholine and CoA from acetyl CoA and choline, and is abbreviated as ChAT.

An "inhibitor of choline acetyltransferase" refers to a substance which inhibits the generation of acetylcholine that is catalyzed by choline acetyltransferase. Examples of inhibitors of choline acetyltransferase include, but are not limited to, compounds disclosed in J. Med. Chem., 1969, vol. 12, 134-38, antibodies that can specifically bind to choline acetyltransferase, and the like.

As used herein, "tertiary amino group" refers to a functional group having the structure of R₃N⁺-. R refers to a substituent. Examples of substituents include alkyl group, alkenyl group, and alkynyl group.

As used herein, "hydrocarbon chain" refers to an alkyl chain, alkenyl chain, or alkynyl chain with one or more carbons. A hydrocarbon chain can be straight or branched.

As used herein, "alkyl" refers to a saturated aliphatic hydrocarbon with one or more carbon atoms.

As used herein, "alkenyl" refers to an aliphatic hydrocarbon with one or more carbon atoms and at least one double bond.

As used herein, "alkynyl" refers to an aliphatic carbon group with one or more carbon atoms and at least one triple bond.

"Analog" refers to a compound, which is structurally similar to the parent compound but has a slightly different composition (e.g., one atom is replaced with an atom of a different element, one functional group is replaced with another functional group in the presence of a specific functional group, or the like). Therefore, analogs are compounds, which have a similar or the same function or outer appearance to the reference parent compound but have a different structure or origin.

"Mimetic" means a molecule retaining a molecular chemical structure required for the functional activity of the original molecule, but comprises a certain chemical structure that is different from the original molecule.

"Derivative" refers to a chemically or biologically modified chemical compound or a small-molecule inhibitor, which is structurally similar to the parent compound and can be (actually or theoretically) derived from the parent compound. "Derivatives" are different from "mimetics" or "functional mimetics" in that a parent compound can be a starting material for preparing a "derivative". A parent compound does not need to be used as a starting material for preparing a "mimetic" or "functional mimetic". A derivative may or may not have a chemical or physical property that is different from those of the parent compound. For example, a derivative can have higher hydrophilicity, or a derivative can have reactivity that is different from that of the parent compound. Derivatization (i.e., modification by chemical or other means) can involve substitutions at one or more portions within a molecule (e.g., a change within a function group). For example, hydrogen may be substituted with halogen such as chlorine or fluorine, or a hydroxyl group (-OH) with a carboxylic acid moiety (-COOH). The term "derivative" also includes conjugates of the parent compound and prodrugs (i.e., a chemically modified derivative, which can be converted into the original compound under physiological conditions). For example, prodrugs can be an inactive form of an active agent. Prodrugs can be converted into an active form of a compound under physiological conditions. Prodrugs can be formed, for example, by substituting one or two hydrogen atoms on a nitrogen atom with an acyl group (acryl prodrug) or a carbamate group (carbamate prodrug). Detailed information regarding prodrugs can be found in, for example, Fleisher et al., Advanced Drug Delivery Reviews 19 (1996) 115; Design of Prodrugs, H. Bundgaard (Ed.), Elsevier, 1985; and H. Bundgaard, Drugs of the Future 16 (1991) 443. The term "derivative" is also used to explain all solvates of the parent compound such as hydrates and adducts (e.g., alcohol adduct), active metabolites, and salts. Types of salts that can be prepared are dependent on the property of the portion within a compound. For example, an acidic group such as a carboxylic group can form an alkali metal salt or alkaline earth metal salt (e.g., sodium salt, potassium salt, magnesium salt, and calcium salt), and salt with a physiologically acceptable quaternary ammonium ion, and acid addition salt of an organic amine such as physiologically acceptable trimethylamine, ethanolamine, or tris-(2-hydroxyethyl)amine and ammonium. A basic group can form an acid addition salt with an inorganic salt such as hydrochloric acid ("HCl"), sulfuric acid, or phosphoric acid, or an organic carboxylic acid and acetic acid, citric acid, benzoic acid, maleic acid, fumaric acid, tartaric acid, methanesulfonic acid, p-toluenesulfonic acid, or other sulfonic acid. A compound simultaneously comprising a basic group and an acidic group, such as a basic nitrogen atom in addition to a carboxyl group, can exist as a zwitterion. A salt can be obtained by a conventional method known to those skilled in the art such as by mixing a compound with an inorganic or organic acid or base in a diluent or solvent, or by cation exchange or anion exchange from another salt.

A "subject" refers to target of administration of the composition or combination or the method for treatment or prevention of the invention. Examples of subjects include mammals (e.g., human, mouse, rat, hamster, rabbit, cat, dog, cow, horse, sheep, monkey, goat, pig, and the like).

### (Preferred embodiment)

The preferred embodiments of the present invention are explained hereinafter. It is understood that the embodiments are exemplification of the present invention, so that the scope of the present invention should not be limited by such preferred embodiments. It is understood that those skilled in the art can refer to the following preferred embodiments to readily make appropriate modifications and changes within the scope of the present invention. Any of these embodiments can be appropriately combined by those skilled in the art.

The present invention provides a composition for treating or preventing atopic dermatitis, comprising an inhibitor of acetylcholine. In one embodiment, inhibitors of acetylcholine include, but are not limited to, an anti-choline agent, acetylcholinesterase, a nucleic acid encoding acetylcholinesterase, an antibody blocking the binding of acetylcholine to an acetylcholine receptor, a nucleic acid encoding an antibody blocking the binding of acetylcholine to an acetylcholine receptor, an antibody to an acetylcholine receptor, a nucleic acid encoding an antibody to an acetylcholine receptor, an agent inhibiting the gene expression of an acetylcholine receptor encoding gene (e.g., ribozyme, antisense nucleic acid, and siRNA to a gene), and an inhibitor of choline acetyltransferase, typically an agent inhibiting the gene expression of choline transferase (e.g., ribozyme, antisense nucleic acid, and siRNA to a gene). Preferably, the inhibitor of acetylcholine is free of crystal violet. The present invention has been completed by newly discovering that elevation in the acetylcholine concentration is a factor for developing atopic dermatitis. Since the mechanism of developing atopic dermatitis had not been elucidated, it was unexpected that inhibition of the action of acetylcholine can effectively treat atopic dermatitis.

The epidermis serves the role of protecting the body from physical or chemical damage, infection, dehydration, heat dissipation, and the like. To accomplish this role, stratum corneum, which is a semitransparent barrier, is formed. To maintain such an important barrier, the epidermis repeats regeneration and repair. Keratinocytes undergo final differentiation and express a series of structural proteins, keratin, or the like. They form fiber to maintain completeness of cells or tissue in the stratum corneum. A ceramide constituting a majority of intracellular lipid is synthesized in the process of keratinocyte keratinization. A ceramide serves the role of retaining moisture in the stratum corneum, constituting one of the barrier functions of the skin. Cell differentiation or barrier formation in the epidermis can be affected by various factors. In recent years, the extraneural cholinergic system has been revealed to be an important factor in the physiological function in the epidermis. Human keratinocytes and endothelial cells are known to synthesize and decompose acetylcholine. Acetylcholine functions through a muscarinic acetylcholine receptor and a nicotinic acetylcholine receptor and is autocrined or paracrined in the surrounding cells. Although not wishing to be bound by any theory, the inventors have elucidated that the acetylcholine concentration is elevated in the skin of atopic dermatitis patients, resulting in acetylcholine receptors to always be activated which leads to an abnormality in the differentiation of keratinocytes. Less cell apoptosis is induced due to the abnormality in the differentiation of keratinocytes, which leads to reduction in ceramide within the stratum corneum. As a result, the barrier function of the skin deteriorates so that the skin is more susceptible to stimulation, leading to repeated scratching that develops atopic dermatitis.

### (Composition comprising anti-choline agent)

In one embodiment, the present invention provides a composition for treating or preventing atopic dermatitis, comprising an anti-choline agent. Alternatively, the present invention provides a method of treating or preventing atopic dermatitis, comprising administering an anti-choline agent.

In one embodiment, the anti-choline agent is an antagonist of an acetylcholine receptor. Acetylcholine receptors are classified into muscarinic acetylcholine receptors and nicotinic acetylcholine receptors. Examples of antagonists of muscarinic acetylcholine receptors include, but are not limited to, trihexyphenidyl hydrochloride, scopolamine butylbromide, pirenzepine hydrochloride hydrate, ipratropium bromide, oxitropium bromide, tiotropium bromide, atropine sulfate, tropicamide, diphenhydramine hydrochloride, dicycloverine, oxybutynin hydrochloride, cyclopentrate hydrochloride, tolterodine tartrate, solifenacin succinate, darifenacin hydrobromide, mevevelin hydrochloride, procyclidine hydrochloride, acridinium bromide, propantheline bromide, scopolamine hydrobromide hydrate, metoscopolamine bromide, mebenzolate bromide, methanethelinium bromide, orphenadrine citrate, homatropine hydrobromide, prefinium bromide, methixene hydrochloride, pipethanate ethobromide, adiphenin hydrochloride, mazaticol hydrochloride hydrate, eutropin chloride, imidafenacin, and fesoterodine fumarate. Examples for nicotinic acetylcholine receptors include, but are not limited to, suxamethonium chloride hydrate, decamethonium bromide, vecuronium bromide, pancuronium bromide, d-tubocurarine chloride hydrochloride hydrate, gallamintriethiodide, mecamylamine hydrochloride, trimetaphan camsilate, hexamethonium bromide, atracurium besilate, doxacurium chloride, mivacurium chloride, 18-methoxycoronaridine, dextromethorphan hydrobromide hydrate, methyllycaconitine, α-bungarotoxin, α-conotoxin G1, benzoquinonium, and bPiDDB.

In one embodiment, the antagonist of an acetylcholine receptor is selected from the group consisting of solifenacin succinate, acridinium bromide, scopolamine hydrobromide hydrate, d-tubocurarine chloride hydrochloride hydrate, gallamintriethiodide, hexamethonium bromide, dextromethorphan hydrobromide hydrate, and α-bungarotoxin.

In another embodiment, the anti-choline agent is a partial agonist or a reverse agonist of an acetylcholine receptor. Agonists are generally substances exhibiting the same action as a biomolecule by binding to a receptor, but a partial agonist exhibiting partial physiological action relative to the original physiological action or an inverse agonist exhibiting the opposite action relative to the original physiological action can be used in the composition of the invention because the original physiological action of the biomolecule can be suppressed. Examples of partial agonists include, but are not limited to, tetramethylammonium bromide (TAB), hexyltrimethylammonium bromide (Hexyl-TAB), sabcomeline, milameline, and the like. Examples of inverse agonists include, but are not limited to, pirenzepine.

It is possible to determine whether a substance can be used as an inhibitor of acetylcholine in the composition of the invention by measuring the anti-choline action. For example, when an antibody to an acetylcholine receptor or a substance inhibiting transcription of a gene encoding an acetylcholine receptor (e.g., ribozyme, antisense nucleic acid, or siRNA to the gene encoding an acetylcholine receptor) is prepared, it is possible to determine whether the prepared substance can be used in the composition of the invention by measuring the anti-choline action. Substances other than the anti-choline agents listed above can also be used in the composition of the invention if the substance potentially having anti-choline action in view of the structural similarity with such anti-choline agents (e.g., analog, mimetics, derivative, and the like) has anti-choline action when the anti-choline action thereof is measured.

The primary screening of substances with anti-choline action can be performed by measuring the interaction with an acetylcholine receptor using Biacore. For example, it is possible to immobilize an acetylcholine receptor to a flow cell and allow a sample to flow into the flow cell to screen a substance binding to the acetylcholine receptor with changes in a sensorgram as an indicator (Spurny et al., Proc Natl Acad Sci U S A. 2015 May 12; 112(19): E2543-52). Candidate substances isolated by the primary screening can be determined as to whether the substances have anti-choline action by measuring the decrease in acetylcholine activity upon adding the candidate substances to a suspension of guinea pig ileum (Acred et al., Br J Pharmacol Chemother. 1957 Dec; 12(4): 447-52.)

In another embodiment, an anti-choline agent is a compound having a structure comprising one or more tertiary amino groups and a single hydrocarbon chain binding thereto. The preferred number of tertiary amino groups is 1 to 2 and more preferably 2. When there are two tertiary amino groups within a molecule, the amino groups are preferably at both ends of the hydrocarbon chain. The preferred number of carbons of a hydrocarbon chain is 1 to 9 and more preferably 1 to 6. A hydrocarbon chain is a straight or branched alkyl chain, alkenyl chain, or alkynyl chain, but is preferably a straight alkyl chain. In a specific embodiment, a substituent on nitrogen of a tertiary amino group is an alkyl group. In yet another embodiment, at least one of the substituents on nitrogen of a tertiary amino group is a methyl group, preferably two are methyl groups, and most preferably three are methyl groups. Nitrogen of a tertiary amino group has a positive charge. Examples of couterions thereof include, but are not limited to, hydroxide ions, fluoride ions, chloride ions, bromide ions, iodide ions, and the like. A counter ion is preferably a bromide ion. It is possible to determine whether these compounds have anti-choline action by well-known methods described above.

An exemplary structure of the compound with one tertiary amino group within a molecule is shown below. 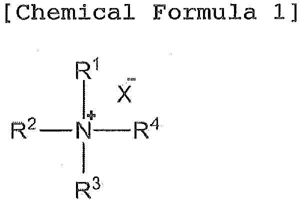 wherein
R¹, R², and R³ are alkyl groups with one or more carbons, and preferably R¹, R², and R³ are each CH₃,
R⁴ is an alkyl chain with 1 to 9 carbons, preferably an alkyl group with 1 to 6 carbons, and
X⁻ is selected from the group consisting of OH⁻, F⁻, Cl⁻, Br⁻, and I⁻.

An exemplary structure of the compound with two tertiary amino groups within a molecule is shown below. 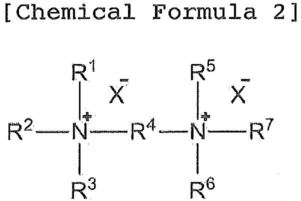 wherein
R¹, R², and R³ are alkyl groups with one or more carbons, and preferably R¹, R², and R³ are each CH₃,
R⁴ is an alkyl chain with 1 to 9 carbons, preferably an alkyl group with 1 to 6 carbons,
X⁻ is selected from the group consisting of OH⁻, F⁻, Cl⁻, Br⁻, and I⁻, and
R⁵, R⁶, and R⁷ are alkyl groups with one or more carbons, and preferably R⁵, R⁶, and R⁷ are each CH₃.

In one embodiment, examples of routes of adminisration of a composition comprising an anti-choline substance include, but are not limited to, topical application, transdermal administration, intradermal administration, subcutaneous administration, intravenous administration, oral administration, enteral administration, pulmonary administration, transnasal administration, intraperitoneal administration, ear drops, eye drops, and the like. As demonstrated in the Examples, the composition of the invention can exert a therapeutic effect independent of the route of administration. Those skilled in the art understand that the composition of the invention can be administered via any route of administration. In some embodiments, a composition is administered by topical application or transdermal administration. Examples of forms of composition for topical application or transdermal administration include, but are not limited to, ointment, poultice, liquid agent, emulsion, spray, paste, cream, lotion, gel, powder, oil, foam, and the like. The anti-choline substance content in a composition for topical application or transdermal administration can be appropriately determined by those skilled in the art depending on the type and toxicity of the anti-choline substance, form of the composition, and dosage. Specific concentrations of an anti-choline substance in a topically applied composition are provided, but are not limited to the following. In a specific embodiment, an anti-choline substance in a topically applied composition is contained, for example, at a concentration of about 0.001 to about 20% by weight. If the anti-choline substance content is too low, atopic dermatitis cannot be sufficiently suppressed in some cases. If the anti-choline substance content is too high, this can lead to toxicity or difficulty in formulation in some cases.

In yet another embodiment, a composition for topical application or transdermal administration can comprise an agent that can promote transdermal absorption or an external use substrate that can promote transdermal absorption in addition to an anti-cholinergic substance. Examples of an agent that can promote transdermal absorption include surfactants such as polyoxyethylene ether and sodium lauryl sulfate, alcohols such as oleyl alcohol, sorbitan esters such as sorbitan monolaurate, cyclic monoterpenes such as I-menthol, urea, and the like. The composition of the invention can comprise an anti-inflammatory agent, steroidal agent, antihistamine agent, preservative, antioxidant, humectant, vitamins, or the like. Examples of anti-inflammatory agent include, but are not limited to, glycyrrhetinic acid, dipotassium glycyrrhizinate, tranexamic acid, allantoin, ε-aminocaproic acid, and the like.

In another embodiment, the composition of the invention can be administered by systemic administration such as oral administration or intravenous administration (but not limited thereto). Example 8 has elucidated that the composition of the invention also exerts a therapeutic effect when administered by oral administration as one example of systemic administration. Those skilled in the art understand that the composition can be administered via any route of administration that is not limited to oral administration, as long as systemic administration is achieved. Systemic administration refers to a route of administration that can distribute the administered compound to the entire body to be available systemically. Examples of dosage forms for oral administration include, but are not limited to, solid or liquid dosage forms, specifically tablets (including sugar-coated tablets and film-coated tablets), pills, granules, powder, capsules (including soft capsules), syrup, emulsion, suspension, and the like. Such dosage forms are manufactured by a known method and can contain a carrier, diluent, or excipient that is commonly used in the field of formulations. Examples of carriers and excipients for tablets include, but are not limited to lactose, starch, saccharose, magnesium stearate, and the like.

For intravenous administration, a carrier can be a solvent or dispersion medium containing, for example, water, ethanol, polyol (e.g., glycerol, propylene glycol, liquid polyethylene glycol, or the like), or a mixture thereof. A composition that is suitable for use in injection of the invention can be a sterilized aqueous solution or dispersion, or sterilized powder for extemporaneous preparation of a sterilized injection solution or dispersion. Action of microorganisms can be prevented with various antimicrobial agents and antifungal agents such as paraben, chlorobutanol, phenol, ascorbic acid, and thimerosal. It is preferable to include an isotonizing agent such as sugar, multivalent alcohol such as mannitol or sorbitol, or sodium chloride in the composition.

The dosage of an anti-choline substance that is orally or intravenously administered can vary depending on the subject of administration, type of substance to be administered, severity, dosage form, or the like, but those skilled in the art can appropriately determine a suitable dosage.

In another embodiment, an inhibitor of acetylcholine is an antibody to an acetylcholine receptor. Examples of antibodies to an acetylcholine receptor include human monoclonal antibodies and humanized monoclonal antibodies (and (F(ab)₂ fragment, Fv fragment, and single chain antibody) that block the binding of acetylcholine to an acetylcholine receptor. Antibodies can be chimerized or humanized. A chimerized antibody herein comprises a constant region of a human antibody and a variable region of a non-human antibody such as a mouse antibody. A humanized antibody comprises a constant region and a framework variable region (i.e., variable region other than the hypervariable region) of a human antibody and a hypervariable region of a non-human antibody such as a mouse antibody. In yet another embodiment, an antibody can be an antibody selected from a phage display system or obtained by any other approach such as a human antibody produced from a Xenomouse or an antibody derivative thereof.

In yet another embodiment, an inhibitor of acetylcholine is an agent inhibiting the gene expression of an acetylcholine receptor. An agent inhibiting the gene expression of an acetylcholine receptor can be used as the active ingredient of the composition of the invention. Examples of an agent inhibiting the gene expression of an acetylcholine receptor include ribozymes, antisense nucleic acids, and siRNA to a gene encoding an acetylcholine receptor, but siRNA is preferred. A siRNA to a gene encoding an acetylcholine receptor can be used to knock down (suppress) the expression thereof.

Design and manufacture of such siRNA and mutant siRNA discussed below are within the technical competence of those skilled in the art. Those skilled in the art can appropriately select any continuous RNA regions of mRNA, which is a transcription product of the sequence of an acetylcholine receptor, to prepare a double stranded RNA corresponding to this region within the normal routine. Those skilled in the art can also appropriately select a siRNA sequence with a more potent RNAi effect from the mRNA sequence, which is a transcription product of the sequence, by a known method.

For the preparation of siRNA, conditions such as (1) no 4 or more consecutive G or C, (2) no 4 or more consecutive A or T, or (3) less than 9 bases of G or C can be added. The length of a double stranded RNA portion, as bases, is 15 to 40 bases, preferably 15 to 30 bases, more preferably 15 to 25 bases, still more preferably 18 to 23 bases, and most preferably 19 to 21 bases. It is understood that the upper and lower limits thereof are not limited to such specific numbers, but can be any combination of the listed numbers. The terminal structure of a sense or antisense strand of siRNA is not particularly limited, but can be appropriately selected depending on the purpose. For example, the structure can have a blunt end or a cohesive end (overhang), and a type with a protruding 3' terminus is preferred. A siRNA with an overhang consisting of several bases, preferably 1 to 3 bases, and more preferably 2 bases at the 3' terminus of a sense RNA strand and an antisense RNA strand is preferred for often having a significant effect of suppressing the expression of a target gene. The type of bases of an overhang is not particularly limited, which can be either a base constituting an RNA or a base constituting a DNA. Examples of a preferred overhang sequence include dTdT (2 bp of deoxy T) at the 3' end and the like. Examples of preferred siRNA include, but are not limited to, siRNA with dTdT (2 bp of deoxy T) at the 3' terminus of all siRNA sense/antisense strands.

Furthermore, it is also possible to use siRNA in which one to several nucleotides are deleted, substituted, inserted and/or added at one or both of the sense strand and antisense strand of the siRNA described above. One to several bases as used herein is not particularly limited, but is preferably 1 to 4 bases, more preferably 1 to 3 bases, and most preferably 1 to 2 bases. Specific examples of such mutations include, but are not limited to, mutations resulting in 0 to 3 bases at the 3' overhang portion, mutations that change the base sequence of the 3'-overhang portion to another base sequence, mutations resulting in the lengths of the sense RNA strand and antisense RNA strand being different by 1 to 3 bases due to insertion, addition or deletion of bases, mutations substituting a base in the sense strand and/or antisense strand with another base, and the like. However, it is necessary that the sense strand and antisense strand can hybridize in such mutant siRNAs, and these mutant siRNAs have the ability to suppress gene expression that is equivalent to that of siRNAs without any mutations.

SiRNA can also be a molecule with a structure in which one end is closed, such as siRNA with a hairpin structure (Short Hairpin RNA; shRNA). A shRNA is an RNA comprising a sense strand RNA of a specific sequence of a target gene, an antisense strand RNA consisting of a sequence complementary to the sense strand sequence, and a linker sequence for connecting the two strands, wherein the sense strand portion hybridizes with the antisense strand portion to form a double-stranded RNA portion.

In order to make the siRNA according to the present invention, a known method, such as a method using chemical synthesis or a method using a gene recombination technique, can be appropriately used. With a method using synthesis, a double-stranded RNA can be synthesized based on sequence information by using a common method. With a method using a gene recombination technique, a siRNA can be made by constructing an expression vector encoding a sense strand sequence or an antisense strand sequence and introducing the vector into a host cell, and then obtaining each of sense strand RNA and antisense strand RNA produced by transcription. It is also possible to make a desired double-stranded RNA by expressing an shRNA forming a hairpin structure, comprising a sense strand of a specific sequence of a target gene, an antisense strand consisting of a sequence complementary to the sense strand sequence, and a linker sequence for linking the two strands.

For a siRNA, all or part of the nucleic acid constituting the siRNA can be a naturally-occurring or modified nucleic acid, as long as such a nucleic acid has activity to suppress the expression of a target gene. A modified nucleic acid refers to a nucleic acid, which has a modification at a nucleoside (base moiety, sugar moiety) and/or an inter-nucleoside binding site and has a structure that is different from that of a naturally occurring nucleic acid.

It is also possible to introduce the nucleic acid or agent of the invention into a phospholipid endoplasmic reticulum such as a liposome (vehicle) and administer the endoplasmic reticulum. An endoplasmic reticulum in which an siRNA or shRNA is retained can be introduced into a given cell using lipofection. The resulting cell is then systemically administered, such as intravenously or intra-arterially. It can also be topically administered to a required site on the skin or the like. While an siRNA exhibits a very good specific, post-transcription suppressing effect in vitro, the siRNA is quickly degraded in vivo due to nuclease activity in the serum, so that the duration thereof is limited. Therefore, there has been a need for the development of a better and more effective delivery system. As an example, Ochiya, T et al., Nature Med., 5: 707-710, 1999, Curr. Gene Ther., 1: 31-52, 2001 reports that a biocompatible material atelocollagen, when mixed with a nucleic acid to form a complex, is a carrier which has an action of protecting a nucleic acid from a degrading enzyme in a living organism and is extremely suitable as a carrier of an siRNA. While such a form can be used, the method for introducing a nucleic acid or medicament of the present invention is not limited thereto. In this manner, it becomes possible to achieve a continued effect for an extended period of time due to the fast degradation by the action of a nuclease in the serum in a living organism. For example, Takeshita F. PNAS, (2003) 102 (34) 12177-82, Minakuchi Y Nucleic Acids Research (2004) 32 (13) e109 report that atelocollagen derived from bovine skin forms a complex with a nucleic acid, which has action of protecting a nucleic acid from a degrading enzyme in a living organism and is extremely suitable as a carrier of an siRNA. Such a technique can be used.

The effect of inhibiting the gene expression of an acetylcholine receptor by an agent inhibiting the gene expression of an acetylcholine receptor can be verified by a well-known method.

In one embodiment, an agent inhibiting the gene expression of an acetylcholine receptor is an antisense nucleic acid. A technology that is well known to those skilled in the art can be used as a method of utilizing an antisense nucleic acid. An antisense nucleic acid can inhibit the expression of a target gene by inhibiting various processes such as transcription, splicing, or translation (Hirashima and Inoue, Shin-seikagaku Jikken Kouza 2 [New Biochemical Experiment Course 2] Kakusan IV Idenshi no Fukusei to Hatsugen [Replication and Expression of Gene of Nucleic Acid IV], Ed. by the Japanese Biochemical Society, Tokyo Kagaku Dojin, 1993, 319-347).

In one embodiment, designing an antisense sequence that is complementary to an untranslated region in the vicinity of the 5' terminus of mRNA of a gene encoding an acetylcholine receptor is considered effective for inhibiting the translation of the gene. A sequence that is complementary to a 3' untranslated region or a coding region can also be used. In this manner, the antisense nucleic acid used in the present invention also encompasses nucleic acids comprising an antisense sequence of the sequence of not only the translated region, but also the untranslated region of a gene encoding an acetylcholine receptor. An antisense nucleic acid to be used is linked downstream of a suitable promoter, and preferably a sequence comprising a transcription termination signal is linked to the 3' side. A nucleic acid prepared in this manner can be transformed into a cell by using a known method. The sequence of an antisense nucleic acid is preferably a sequence that is complementary to a gene encoding an acetylcholine receptor of the cell to be transformed or a portion thereof. However, such a sequence does not need to be fully complementary, as long as the gene expression can be effectively suppressed. A transcribed RNA preferably has complementarity that is 90% or greater, and most preferably 95% or greater, with respect to a transcript of a target gene. In order to effectively inhibit the expression of a target gene using an antisense nucleic acid, it is preferable that the length of the antisense nucleic acid is at least 12 bases and less than 25 bases, but the antisense nucleic acid of the invention is not necessarily limited to this length. For example, the length may be 11 bases or less, 100 bases or more, or 500 bases or more. An antisense nucleic acid may be composed of only DNA, but may comprise a nucleic acid other than DNAs, such as a locked nucleic acid (LNA). As one embodiment, an antisense nucleic acid used in the invention can be an LNA containing antisense nucleic acid comprising LNA at the 5' terminus or LNA at the 3' terminus. In an embodiment using the antisense nucleic acid in the invention, the antisense sequence can be designed using the method described in, for example, Hirashima and Inoue, Shin-seikagaku Jikkenn Kouza 2 [New Biochemical Experiment Course 2] Kakusan IV Idenshi no Fukusei to Hatsugen [Replication and Expression of Gene of Nucleic Acid IV], Ed. by the Japanese Biochemical Society, Tokyo Kagaku Dojin, 1993, 319-347.

In one embodiment, an agent inhibiting the gene expression of an acetylcholine receptor is a ribozyme or DNA encoding a ribozyme. A ribozyme refers to an RNA molecule having catalytic activity. While there are ribozymes with various activities, a study focusing on especially ribozymes as an enzyme for cleaving an RNA made it possible to design a ribozyme that site-specifically cleaves an RNA. There are ribozymes with a size of 400 nucleotides or more as in M1 RNA contained in RNase P and group I intron ribozymes, but there are also those with an active domain of about 40 nucleotides called hammerhead or hair-pin ribozymes (Makoto Koizumi and Eiko Otsuka, Protein, Nucleic Acid and Enzyme, 1990, 35, 2191).

Hairpin ribozymes are also useful for the objective of the invention. Such a ribozyme is found, for example, in the minus strand of a tobacco ringspot virus satellite RNA (Buzayan J M, Nature, 1986, 323, 349). It is demonstrated that target specific RNA-cleaving ribozymes can also be created from hairpin ribozymes (Kikuchi, Y. & Sasaki, N., Nucl. Acids Res, 1991, 19, 6751., Yo Kikuchi, Kagaku to Seibutsu [Chemistry and Biology], 1992, 30, 112). In this manner, expression of a gene encoding an acetylcholine receptor can be inhibited by specifically cleaving a transcript of the gene by using a ribozyme.

In another embodiment, the present invention provides a composition for treating or preventing atopic dermatitis, comprising an inhibitor of choline acetyltransferase. Alternatively, the present invention provides a method of treating or preventing atopic dermatitis, comprising administering an inhibitor of choline acetyltransferase. As discussed above, the inventors have newly discovered that atopic dermatitis is induced by elevation in the acetylcholine concentration. In other words, the action of an enzyme producing acetylcholine can be inhibited to suppress elevation in the acetylcholine concentration. Therefore, an inhibitor of choline acetyltransferase, which is an enzyme generating acetylcholine, can also be used in the composition of the invention.

In one embodiment, an inhibitor of choline acetyltransferase is a compound that inhibits choline acetyltransferase such as 4-[2-(2-naphthyl)vinyl]pyridine or 4-[2-(2-naphthyl)acetinyl]pyridine (Japanese National Phase PCT Laid-open Publication No. 2003-52886, J. Med. Chem., 1969, vol.12, 134-38). In another embodiment, an inhibitor of choline acetyltransferase is an anti-choline acetyltransferase antibody.

In still another embodiment, the present invention provides a composition or combination for treating or preventing atopic dermatitis, comprising an inhibitor of muscarinic acetylcholine and an inhibitor of nicotinic acetylcholine. A high therapeutic effect is expected by suppressing the action of both muscarinic acetylcholine and nicotinic acetylcholine. In one embodiment, the inhibitor of muscarinic acetylcholine can be an antagonist of a muscarinic acetylcholine receptor, and the inhibitor of nicotinic acetylcholine can be an antagonist of a nicotinic acetylcholine receptor. An inhibitor of muscarinic acetylcholine and an inhibitor of nicotinic acetylcholine in a combination can be simultaneously or successively administered.

In still another embodiment, the antagonist of a muscarinic acetylcholine receptor is selected from the group consisting of trihexyphenidyl hydrochloride, scopolamine butylbromide, pirenzepine hydrochloride hydrate, ipratropium bromide, oxitropium bromide, tiotropium bromide, atropine sulfate, tropicamide, diphenhydramine hydrochloride, dicycloverine, oxybutynin hydrochloride, cyclopentrate hydrochloride, tolterodine tartrate, solifenacin succinate, darifenacin hydrobromide, mevevelin hydrochloride, procyclidine hydrochloride, acridinium bromide, propantheline bromide, scopolamine hydrobromide hydrate, metoscopolamine bromide, mebenzolate bromide, methanethelinium bromide, orphenadrine citrate, homatropine hydrobromide, prefinium bromide, methixene hydrochloride, pipethanate ethobromide, adiphenin hydrochloride, mazaticol hydrochloride hydrate, eutropin chloride, imidafenacin, and fesoterodine fumarate.

In still another embodiment, the antagonist of a nicotinic acetylcholine receptor is selected from the group consisting of suxamethonium chloride hydrate, decamethonium bromide, vecuronium bromide, pancuronium bromide, d-tubocurarine chloride hydrochloride hydrate, gallamintriethiodide, mecamylamine hydrochloride, trimetaphan camsilate, hexamethonium bromide, atracurium besilate, doxacurium chloride, mivacurium chloride, 18-methoxycoronaridine, dextromethorphan hydrobromide hydrate, methyllycaconitine, α-bungarotoxin, α-conotoxin G1, benzoquinonium, and bPiDDB.

In a specific embodiment, the antagonist of a muscarinic acetylcholine receptor can be solifenacin succinate, acridinium bromide, or scopolamine hydrobromide hydrate, and the antagonist of a nicotinic acetylcholine receptor can be d-tubocurarine chloride hydrochloride hydrate, gallamintriethiodide, hexamethonium bromide, dextromethorphan hydrobromide hydrate, or α-bungarotoxin. Preferably, the antagonist of a muscarinic acetylcholine receptor is scopolamine hydrobromide hydrate, and the antagonist of a nicotinic acetylcholine receptor is hexamethonium bromide.

In still another embodiment, the present invention provides a method of treating or preventing atopic dermatitis in a subject, comprising administering an inhibitor of acetylcholine to the subject. Inhibitors of acetylcholine have been described above in detail.

In still another embodiment, the present invention provides a method of treating or preventing atopic dermatitis in a subject, comprising administering an inhibitor of muscarinic acetylcholine and an inhibitor of nicotinic acetylcholine to the subject. Inhibitors of muscarinic acetylcholine and inhibitors of nicotinic acetylcholine have been described above in detail as an inhibitor of muscarinic acetylcholine and an inhibitor of nicotinic acetylcholine used in a combination. An inhibitor of muscarinic acetylcholine and an inhibitor of nicotinic acetylcholine can be simultaneously or successively administered.

In still another embodiment, the present invention provides use of an inhibitor of acetylcholine in the manufacture of a medicament for treating or preventing atopic dermatitis in a subject. Inhibitors of acetylcholine have been described above in detail.

Reference literatures such as scientific literatures, patents, and patent applications cited herein are incorporated herein by reference to the same extent that the entirety of each document is specifically described.

The present invention has been explained while showing preferred embodiments to facilitate understanding. The present invention is explained hereinafter based on Examples. The above explanation and the following Examples are not provided to limit the present invention, but for the sole purpose of exemplification. Thus, the scope of the present invention is not limited to the embodiments and Examples that are specifically disclosed herein and is limited only by the scope of claims.

### [Examples]

### (Example 1: Evaluation of drug efficacy in atopic dermatitis model mice)

This Example studied whether an anti-choline drug is an agent that is effective for treating AD diseases by histological analysis and investigation by visual observation using AD scores.

### (Materials and Methods)

### Preparation of atopic dermatitis model mice:

10 week old NC/Nga mice (Charles River Laboratories Japan) were used as atopic dermatitis model animals. A mite (Dermatophagoides farina) antigen, i.e., Biostir AD (Wako Pure Chemical), was used to induce dermatitis. Model mice were prepared by the following procedure.
1. The back of mice was shaved. A hair removal cream was used only for the first time.
2. To destroy the skin barrier, 150 µL of 4% SDS was applied and dried for 2 to 3 hours (this step was omitted for the first time).
3. To stimulate immunity, 100 mg of Biostir AD was applied to the back and the left and right auricles of the mice.
4. These steps were performed twice a week.

Scoring was performed according to the method described in the package insert of Biostir AD. Four endpoints were each evaluated as 0 to 3 points. The total score for the endpoints was used as the AD score. The following Table 1 summarizes the scores for each endpoint.

**[Table 1]**

| **Back: Flares/hemorrhage** | | |
|---|---|---|
| 0: | No symptom | None |
| 1: | Mild | Flares (local) |
| 2: | Moderate | Flares (scattered) |
| 3: | Severe | Flares (whole) or scratch and hemorrhage |

| **Back: Scab/dryness** | | |
|---|---|---|
| 0: | No symptom | None |
| 1: | Mild | Local; turned white; slight detachment of stratum corneum |
| 2: | Moderate | Scattered; clear detachment of stratum corneum |
| 3: | Severe | Whole; clear detachment of stratum corneum |
| | | |

| **Auricle: Edema** | | |
|---|---|---|
| 0: | No symptom | None |
| 1: | Mild | Left or right |
| 2: | Moderate | Both |
| 3: | Severe | Both (warped or hard) |

| **Auricle: Tissue loss** | | |
|---|---|---|
| 0: | No symptom | None |
| 1: | Mild | Non-continuous scratches; no loss |
| 2: | Moderate | Small continuous scratches; no loss |
| 3: | Severe | Continuous scratches with loss |

### Preparation of test formulation:

Acridinium bromide (Wako Pure Chemical) and scopolamine hydrobromide trihydrate (Tokyo Chemical Industry) were used as an inhibitor of a muscarinic acetylcholine receptor. Hexamethonium bromide (sigma-aldrich) and dextromethorphan hydrobromide monohydrate (Wako Pure Chemical) were used as an inhibitor of a nicotinic acetylcholine receptor. Dimethyl sulfoxide (DMSO, Wako Pure Chemical), saline (Otsuka Pharmaceutical), and purified water were used as solvents. The final concentrations and solvents of the test formulations are shown in Table 2.

### [Table 2]

**Table 2 Test formulation**

| Name of substance | Final concentration | Solvent |
|---|---|---|
| Acridinium bromide | 12 mM | Diluted with saline so as to result in 20% DMSO (v/v) |
| Scopolamine hydrobromide trihydrate | 12 mM | Saline |
| Hexamethonium bromide | 12 mM | Saline |
| Dextromethorphan hydro bromide monohydrate | 12 mM | Saline |
| Saline | - | Saline |

### Evaluation of drug efficacy after inducing dermatitis:

Female NC/Nga mice (Charles River Laboratories Japan) induced to have atopic dermatitis were used. A test formulation was applied to the back and left and right auricles once a day, 5 days a week (Monday to Friday), for a total of 5 times. 100 µL of the test formulation was applied to the back, and 25 µL was applied to each of the left and right auricles. The back was shaved prior to administration on Monday and Thursday to evaluate dermatitis scores. Mice were euthanized after 7 days from the initial administration, and then the skin tissue was sampled. The skin samples of the cervical and lumbar region were subjected to various analyses as the severe and mild areas, respectively.

### (Results)

Evaluation of drug efficacy after inducing dermatitis:

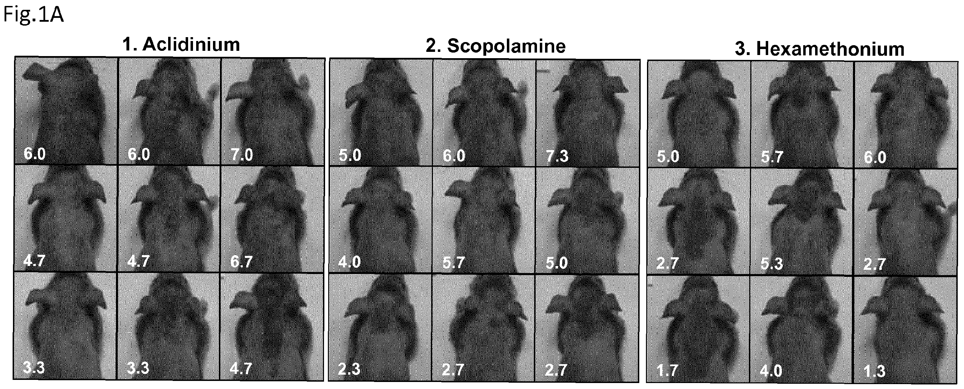

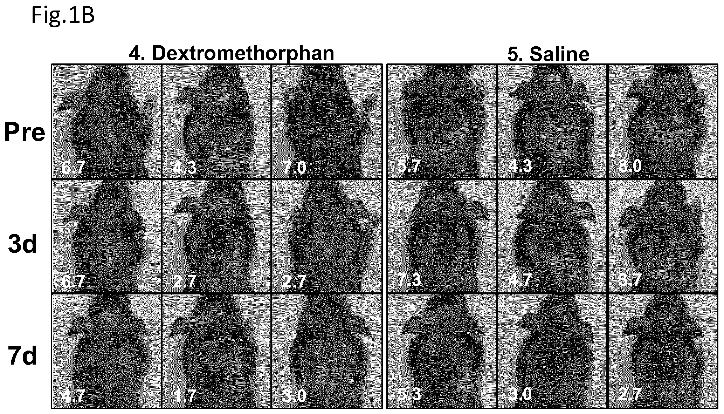

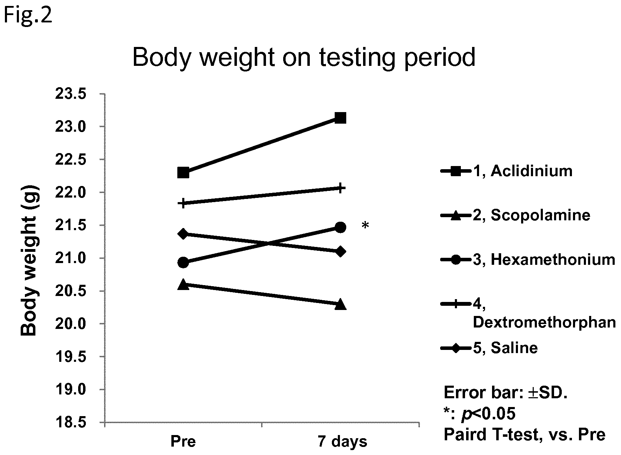

Figure **1A** and **1B** show pictures of the back of the mouse group to which a test formulation has been applied. The skin of mice applied with each test formulation had less damage on the skin, trending to recovery, on day 7 from the start of application. As shown in Figure 2, a significant change in body weight was not observed between before application and day 7 from starting application, suggesting that there is no side effect due to application of a test formulation.

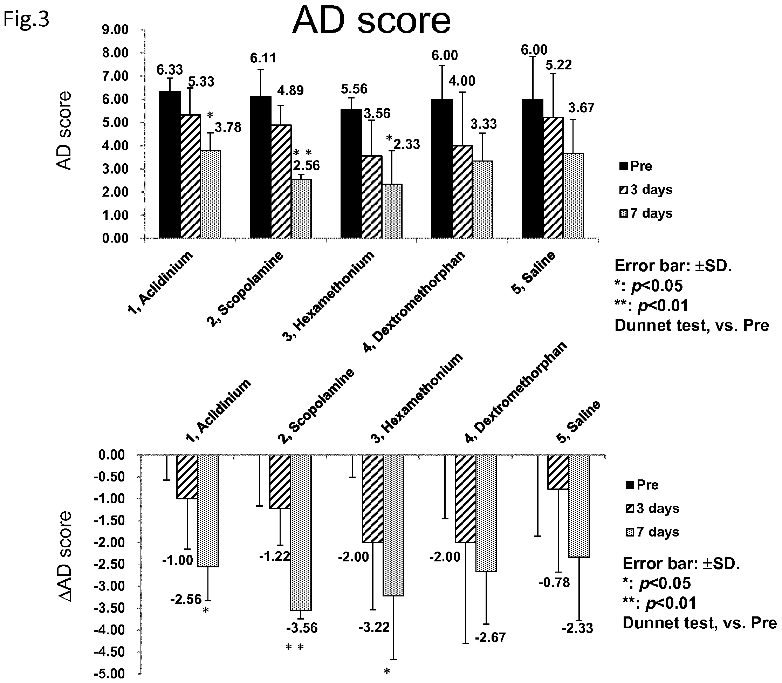

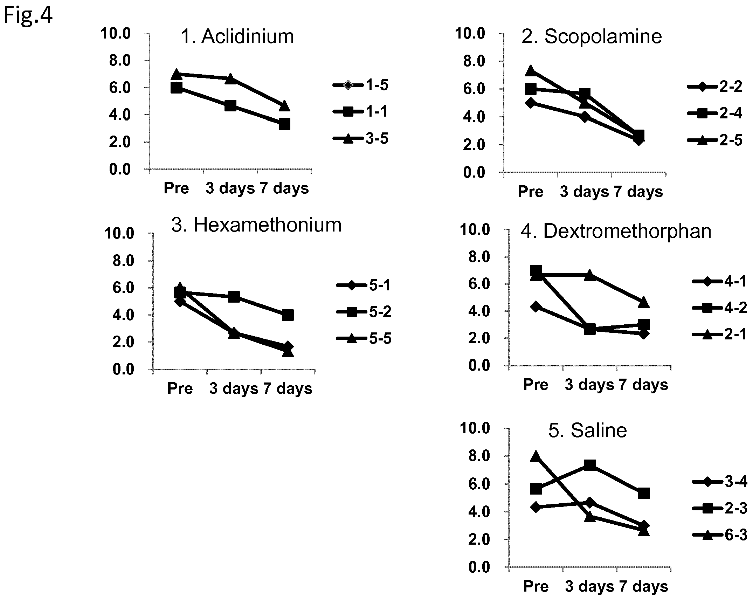

Figure **3** shows a graph that summarizes the results of AD scores and ΔAD scores (difference in AD scores before application and after starting application). Figure 4 shows the change in AD scores of each mouse group applied with each test formulation (horizontal axis indicates the number of days). As shown, hexamethonium bromide had a relatively high therapeutic effect, but the therapeutic effect was not significantly different among all test formulations. This result indicates that all of the inhibitors of acetylcholine are effective in treating atopic dermatitis.

### (Example 2: Evaluation of activity and migration of mast cells)

This Example studied whether there is an effect of suppressing degranulation of mast cells in relation to inflammation or immune response.

### (Materials and Methods)

### Preparation of test formulations:

Solifenacin succinate (Tokyo Chemical Industry), acridinium bromide (Wako Pure Chemical) and scopolamine hydrobromide trihydrate (Tokyo Chemical Industry) were used as an inhibitor of a muscarinic acetylcholine receptor. Tubocurarine chloride pentahydrate (Tokyo Chemical Industry), gallamintriethiodide (sigma-aldrich), hexamethonium bromide (sigma-aldrich), dextromethorphan hydrobromide monohydrate (Wako Pure Chemical), and α-bungarotoxin (alomone labs) were used as an inhibitor of a nicotinic acetylcholine receptor. Dimethyl sulfoxide (DMSO, Wako Pure Chemical) saline (Otsuka Pharmaceutical), and purified water were used as solvents. The final concentrations and solvents of the test formulations are shown in Table 3.

**Table 3 Test formulation**

| Name of substance | Final concentration | Solvent |
|---|---|---|
| Solifenacin succinate | 12 mM | Saline |
| Acridinium bromide | 12 mM | Diluted with saline so as to result in 20% DMSO (v/v) |
| Scopolamine hydro bromide trihydrate | 12 mM | Saline |
| Tubocurarine chloride pentahydrate | 1.2 mM | Saline |
| Gallamintriethiodide | 12 mM | Saline |
| Hexamethonium bromide | 12 mM | Saline |
| Dextromethorphan hydrobromide monohydrate | 12 mM | Saline |
| α-bungarotoxin | 0.12 mM | Saline |
| Saline | - | Saline |

The back skin of sampled atopic dermatitis model mice was immobilized with neutral formalin and embedded in paraffin. A 4 µm thick tissue segment was prepared therefrom, and mast cells were stained with toluidine blue stain. The stained tissue segment was captured with a microscope. Cells stained in dark purple were counted as inactive mast cells (inactive) and cells stained in reddish purple were counted as degranulated mast cells (active). Further, cells exhibiting a medium degree of stain were counted as cells that have been degranulated to a moderate degree (middle). The areas of only collagen fiber sites in the tissue segment images were measured (removing areas of the epidermal layer, mast cells, and muscle cells) and compared the mast cell count per unit area to the degree of degranulation.

### (Results)

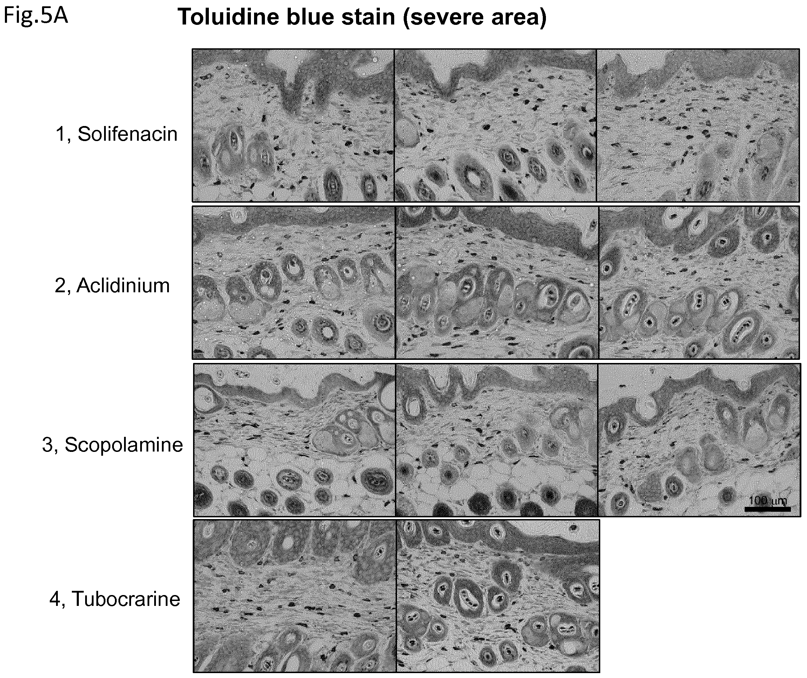

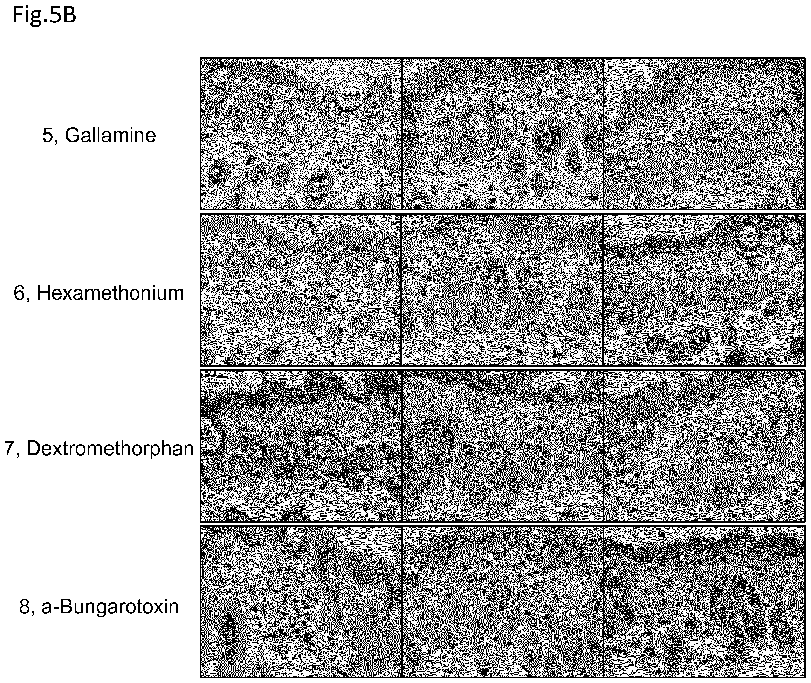

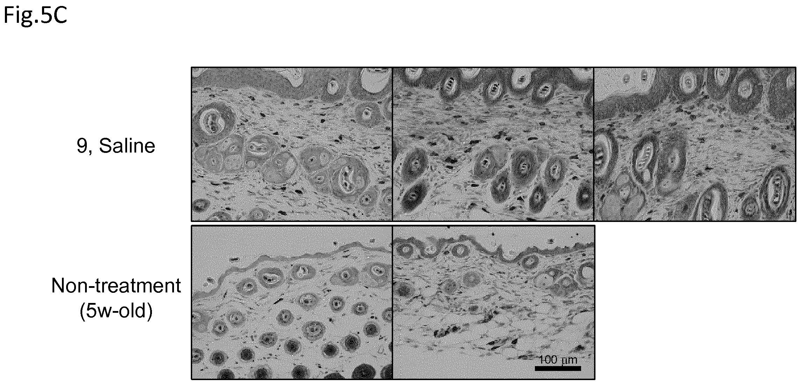

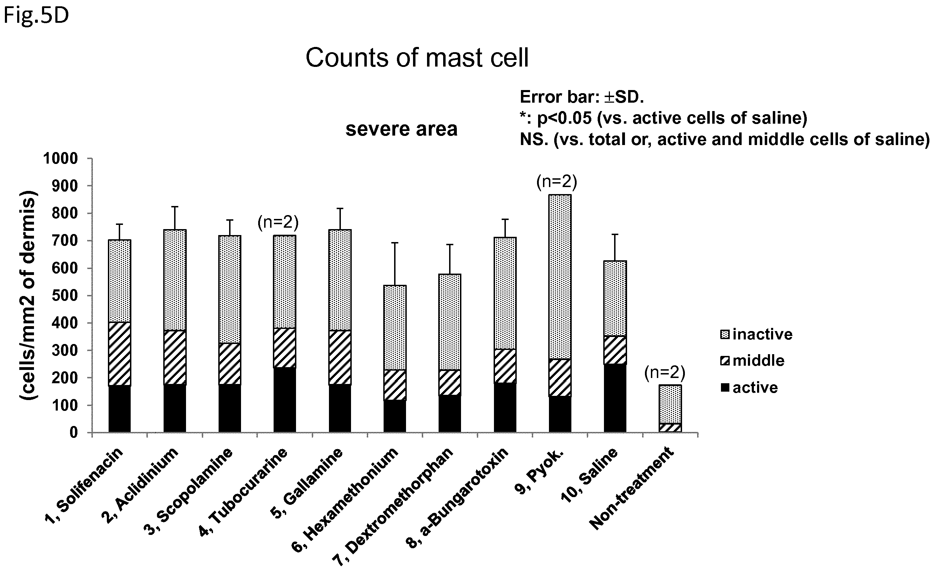

Figures **5A** to **5C** show microscope pictures of tissue segments stained with toluidine blue. Inactive mast cells, degranulated mast cells, and moderately degranulated mast cells were counted based on the degree of staining of the mast cells in the microscope pictures. Figure 5D shows a graph of mast cell count in a tissue segment. In a mouse group without induction of atopic dermatitis (Non-treatment), mast cell count was lower than mice induced with atopic dermatitis (10. Saline), and hardly any degranulated cells were observed. It was found that relative to the severe area of the mouse group applied with saline (10. Saline), the degranulated mast cell (active) count at the severe area of the mouse groups applied with a test formulation was low, whereas non-degranulated mast cell (inactive) count tended to be high. The ratio of degranulated mast cells to all mast cells in the mouse group applied with saline (10. Saline) was about 40%, while the ratio of degranulated mast cells in the mouse groups applied with a test formulation was on average about 25% and at most about 33% in the tubocurarine application group. These results indicate that degranulation was suppressed to a certain degree in mouse groups applied with a test formulation.

### (Example 3: Evaluation of drug efficacy from combined use of drugs and dermatitis induction)

This Example also induced atopic dermatitis during the test formulation application period in parallel to evaluate the drug efficacy of test formulations.

### (Materials and Methods)

Atopic dermatitis model mice were prepared and scored in the same manner as Example 1. The same test formulations as those in Example 1 were used.

Male NC/Nga mice (Charles River Laboratories Japan) induced to have atopic dermatitis were used. A test formulation was applied to the back once a day, 5 days a week (Monday to Friday), for a total of 10 times. 100 µL of the test formulation was applied to the back, and 25 µL was applied to each of the left and right auricles. The back was shaved prior to administration on Monday and Thursday to evaluate dermatitis scores. Atopic dermatitis was then induced only on Mondays every week to maintain dermatitis. Mice were euthanized after 11 days from the initial administration, and then the skin tissue was sampled.

### (Results)

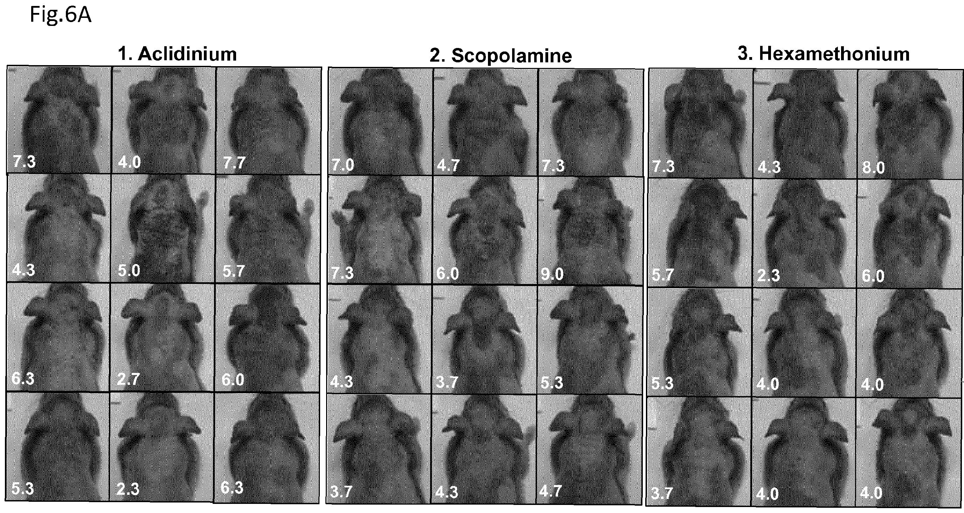

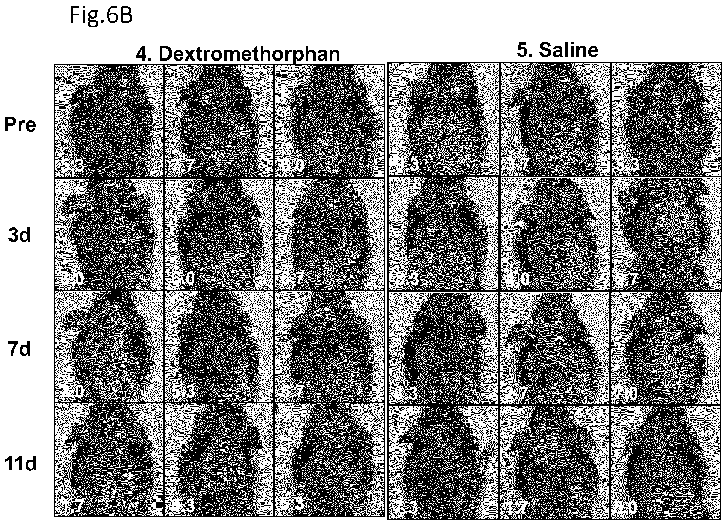

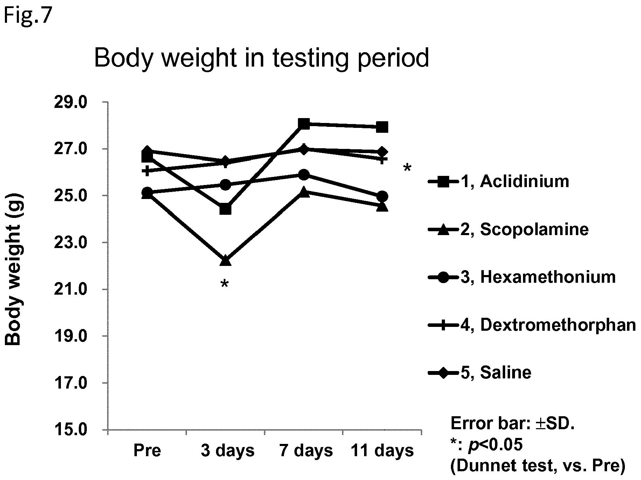

Figures **6A** and **6B** show pictures of the back of the mouse group to which a test formulation has been applied. The skin of mice applied with each test formulation had less damage on the skin, trending to recovery, on day 11 from the start of application despite induction of atopic dermatitis in parallel. As shown in Figure 7, a significant change in body weight was not observed between before application and day 11 from starting application, suggesting that there is no side effect due to application of a test formulation.

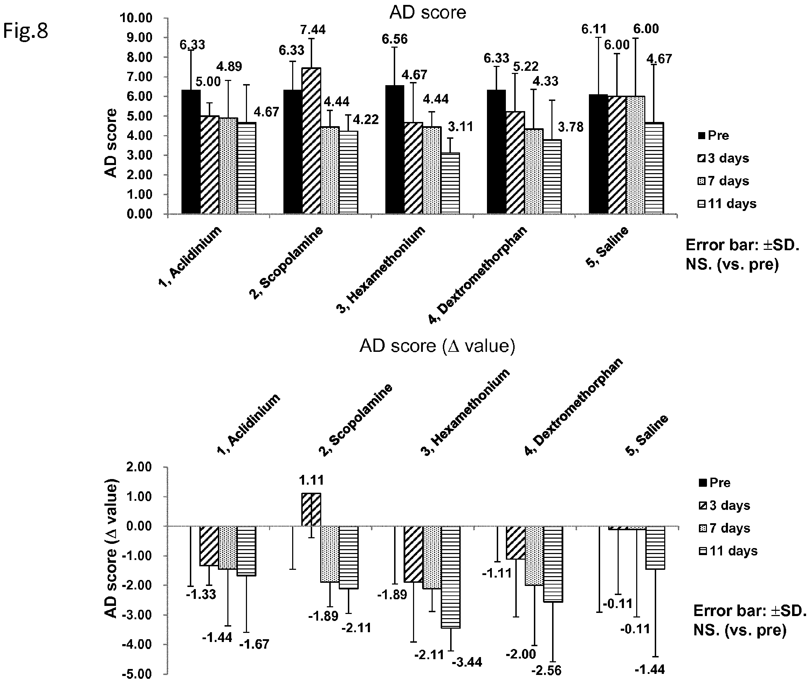

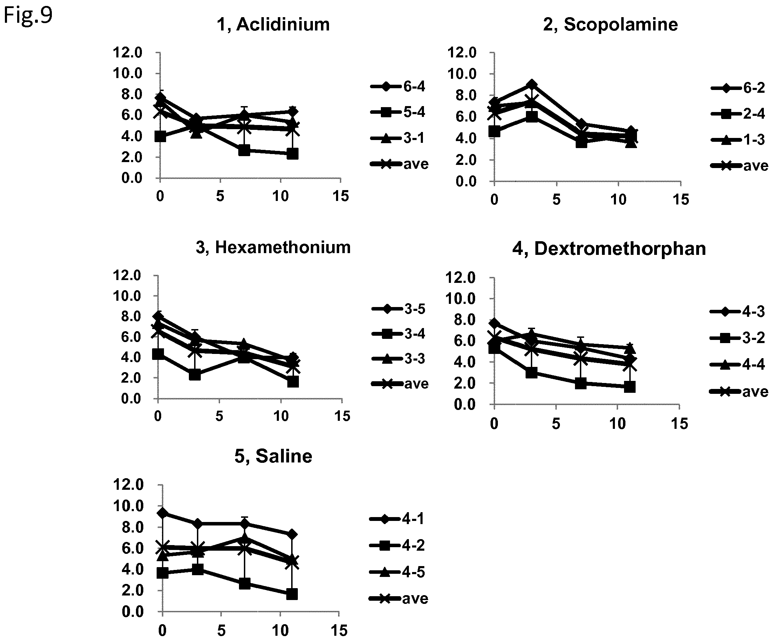

Figure **8** shows a graph that summarizes AD scores and ΔAD scores (difference in AD scores before application and after starting application). Figure 9 shows the change in AD scores of each mouse group applied with each test formulation (horizontal axis indicates the number of days). Hexamethonium bromide had a relative high therapeutic effect, but the therapeutic effect was not significantly different among all test formulations, even with induction of atopic dermatitis in parallel. This result indicates that all of the inhibitors of acetylcholine are effective in treating atopic dermatitis.

### (Example 4: Quantification of acetylcholine concentration in the skin)

This Example demonstrates that the acetylcholine concentration in the skin of mice with atopic dermatitis is elevated.

100 µL of saline was added to 20 mg of thinly sliced mouse back skin tissue and stirred overnight at 4°C, and then the supernatant centrifuged for 15 minutes at 15000 rpm was used as the measurement sample. 50 µL was fractionated from the sample to measure acetylcholine according to the instruction manual of Amplite Fluorimetric Acetylcholine Assay Kit (AAT Bioquest). The acetylcholine concentration in the skin was calculated based on the fluorescence signal value obtained by measurement of the sample and standard curves.

### (Results)

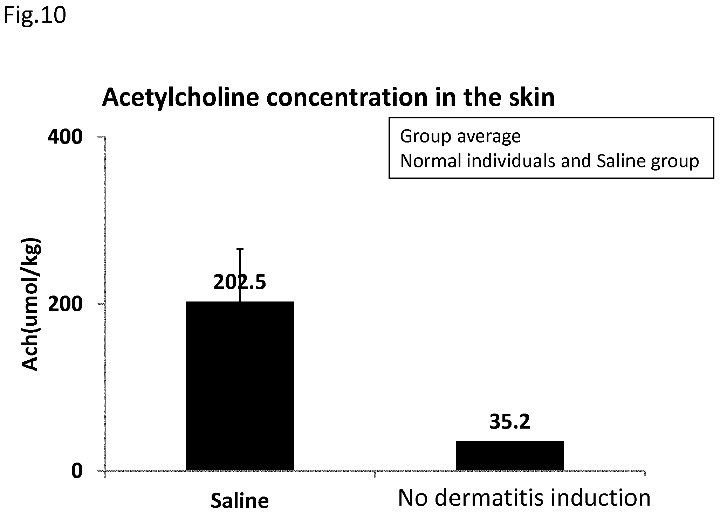

The acetylcholine concentration in the skin was measured and compared using skin samples of a mouse group with atopic dermatitis induction and saline application and a mouse group without atopic dermatitis induction. The results are shown in Figure 10. As shown, acetylcholine in the skin was significantly higher in the mouse group with atopic dermatitis induction relative to the mouse group without atopic dermatitis induction. These results demonstrated that the acetylcholine concentration in the skin is elevated in atopic dermatitis mice relative to normal mice.

### (Example 5: Therapeutic effect of a compound with a molecular structure that is similar to hexamethonium bromide on atopic dermatitis)

The effect of compounds with a molecular structure similar to hexamethonium bromide, which has a relatively high therapeutic effect, on atopic dermatitis therapy due to a difference in agonist and antagonist, amino group class, number of tertiary amino groups, and number of carbon chains was analyzed.

### (Materials and Methods)

Mice (NC/Nga, 10 week old) were applied with a mite antigen (Biostir AD) twice a week for 4 weeks to prepare mice with atopic dermatitis outbreak as in Example 1. Induction with mite antigens (twice a week) and application of a test formulation (5 times a week) were simultaneously performed for 3 weeks to analyze the effect of suppressing induction of atopic dermatitis. A test formulation was used at 12 mM in week 1, and at 24 mM in week 2 and thereafter. However, strong toxicity was found in decamethonium bromide, so that use thereof was changed to 12 mM in week 2 and thereafter and the dosing interval to twice a week in the last week. A non treatment group (no induction, no application of test formulation) and solvent applied group (with induction, application of purified water) were prepared as controls. Scoring was performed in the same manner as Example 1.

### Test formulation:

The structures of the test formulations are shown in Table 4. The therapeutic effect on atopic dermatitis was compared from the viewpoint of the following (1)-(4).
(1) Antagonist and agonist: hexamethonium bromide, suxamethonium chloride (Maruishi Pharmaceutical), and decamethonium bromide (Tokyo Chemical Industry) were used to compare an antagonist and agonist of a nicotinic acetylcholine receptor.
(2) Amino group class: N,N,N',N'-tetramethyl-1,6-hexanediamine (Wako Pure Chemical; hereinafter, referred to as TMHDA) and hexamethylendiamine (Wako Pure Chemical; hereinafter, referred to as HMDA) in which a tertiary amino group of hexamethonium bromide was changed to a secondary amino group and amino group, respectively, were compared.
(3) Number of tertiary amino groups: Hexamethonium bromide has two tertiary amino groups within the molecule. This was compared to hexyltrimethylammonium bromide (Wako Pure Chemical; hereinafter, referred to as Hexyl-TAB) having one tertiary amino group within the molecule and an alkyl group with 6 carbons.
(4) Carbon chain length: Hexamethonium bromide was compared with decamethonium bromide (number of carbons 10) which has a carbon chain length that is longer than hexamethonium bromide (number of carbons 6), and compared with tetramethylammonium bromide (Wako Pure Chemical; number of carbons: 1; hereinafter, referred to as TAB), hexyltrimethylammonium bromide (number of carbons: 6; hereinafter, referred to as Hexyl-TAB), and cetyltrimethylammonium bromide (Wako Pure Chemical; number of carbons: 16; hereinafter, referred to as Cetyl-TAB), which have one tertiary amino group within the molecule and a different carbon chain length in the alkyl group.

Each formulation was dissolved in purified water and subjected to a test at a final concentration of 12 or 24 mM.

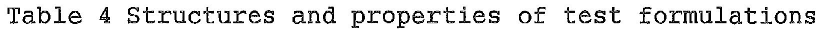

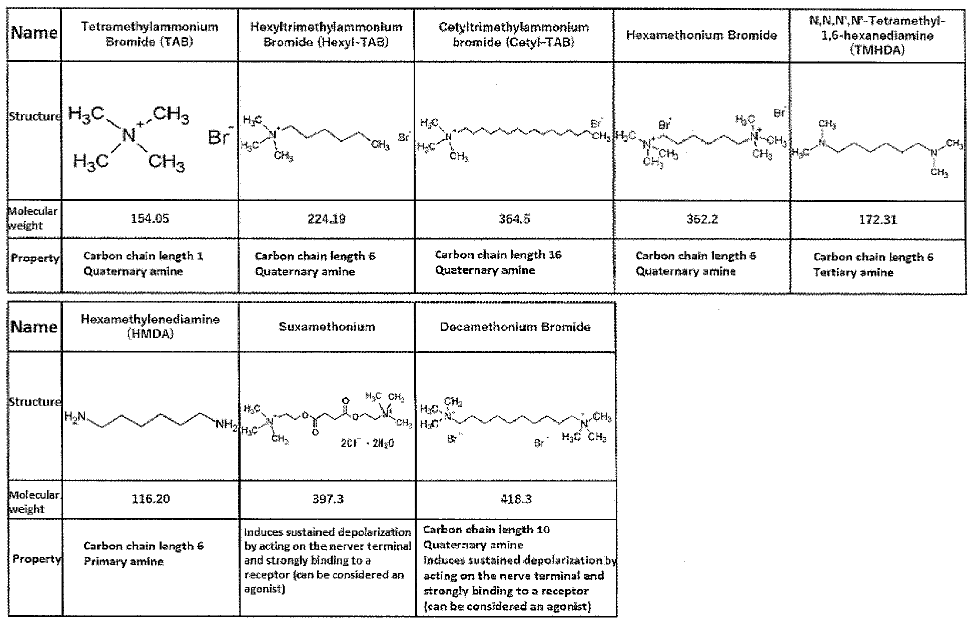

### (Results)

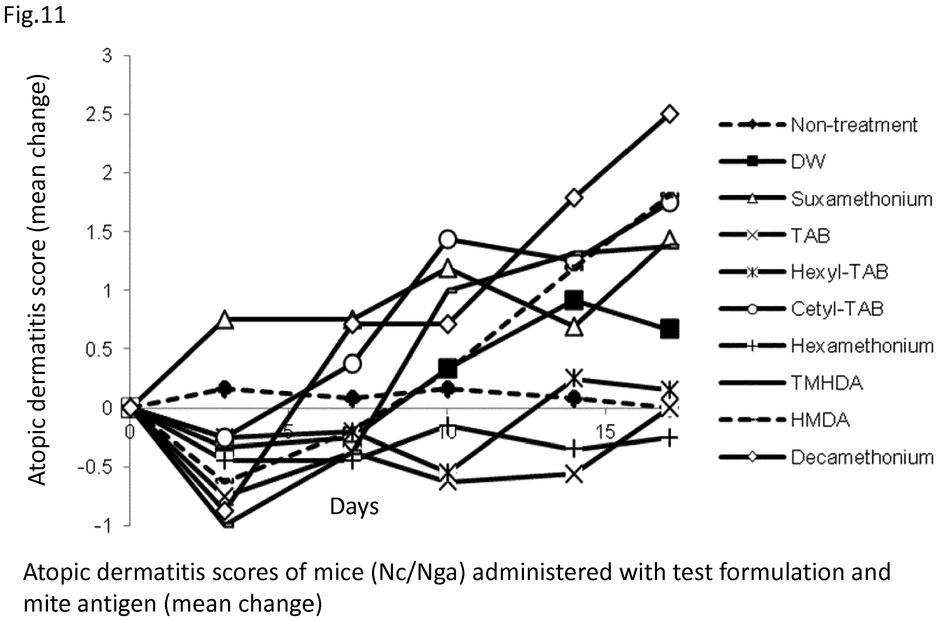

Figure **11** shows the mean change in atopic dermatitis scores during the test formulation application period.

### (1) Comparison of antagonist with agonist

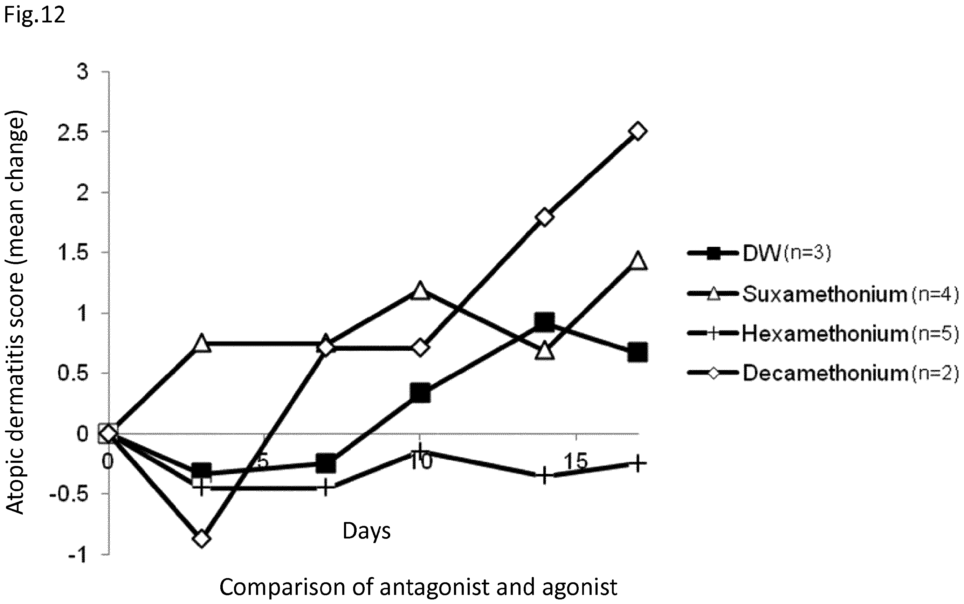

Each of hexamethonium bromide, which is an antagonist of a nicotinic acetylcholine receptor, and suxamethonium chloride and decamethonium bromide, which are agonist, was administered to atopic dermatitis model mice to compare dermatitis scores. Figure **12** shows the mean change in atopic dermatitis scores during the test formulation application period. Suxamethonium bromide and decamethonium bromide were confirmed to reduce atopic dermatitis scores compared to the solvent administered group (DW). Meanwhile, elevation in atopic dermatitis scores was suppressed in the group administered with an antagonist hexamethonium bromide relative to the solvent administered group. It is understood that suxamethonium bromide and decamethonium bromide resulted in reduced atopic dermatitis scores because suxamethonium bromide and decamethonium bromide are agonists that excessively activate a receptor more than acetylcholine in the body. The above result demonstrates that an agonist of a nicotinic acetylcholine receptor does not have an effect of treating atopic dermatitis. It was also confirmed that exacerbation of atopic dermatitis can be suppressed by suppressing the activity of a nicotinic acetylcholine receptor with an antagonist.

### (2) Comparison of amino group classes

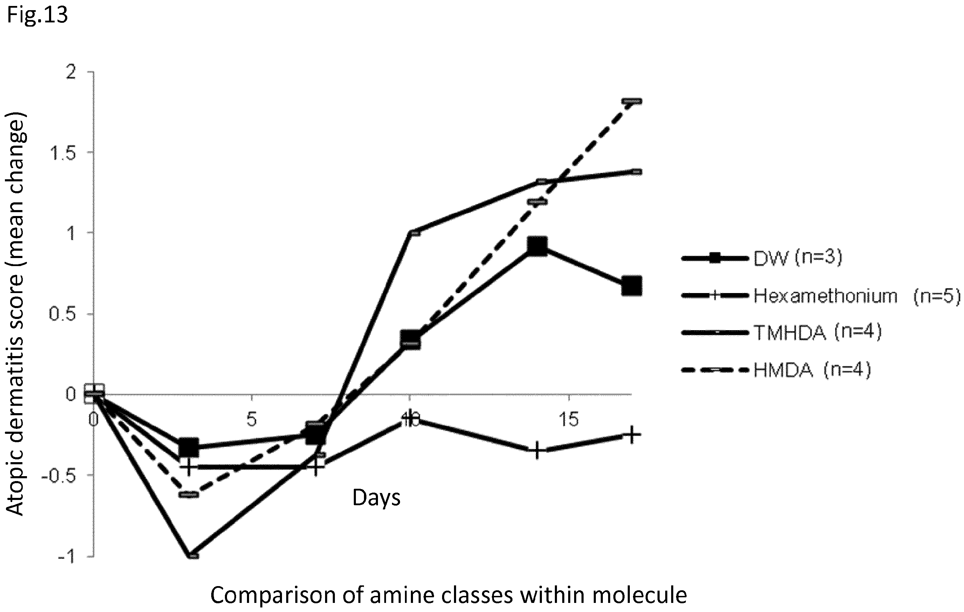

Hexamethonium bromide has two tertiary amino groups within the molecule. In this regard, this was compared with TMHDA and HMDA in which the amino group is a tertiary or primary amino group. Figure 13 shows the results. An effect of suppression was not found in both TMHDA and HMDA. The above results suggest the possibility that a compound with a tertiary amino group is effective in suppressing atopic dermatitis.

### (3) Comparison of number of tertiary amino groups within the molecule

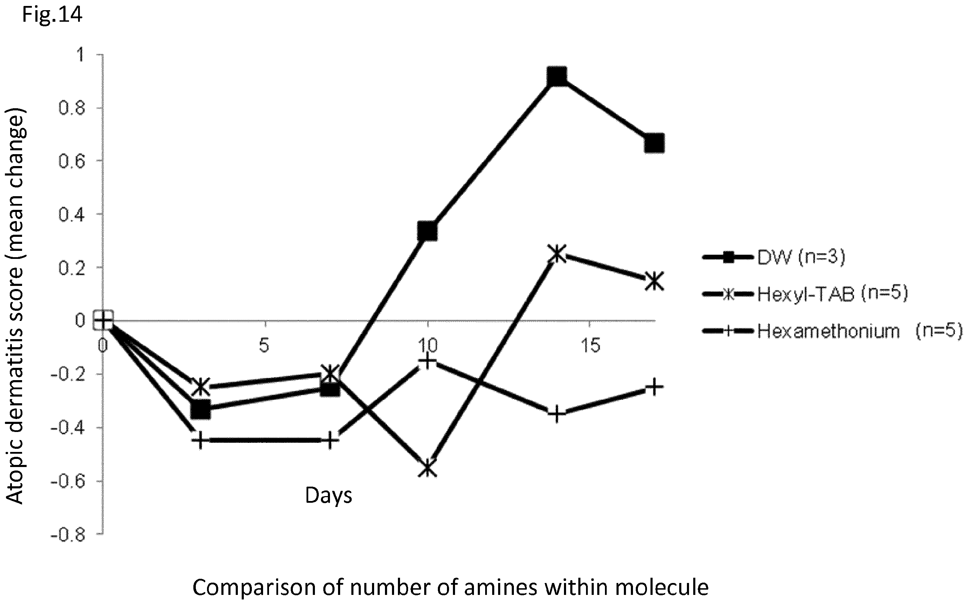

Hexyl-TAB with one tertiary amino group within the molecule was compared to hexamethonium bromide with two tertiary amino groups. Figure **14** shows the result thereof. Hexyl-TAB was also confirmed to have an effect of suppressing atopic dermatitis, although slightly weaker than hexamethonium bromide. The result suggests that any tertiary amine would have an effect of suppressing atopic dermatitis, and greater number of amino groups within the molecule would have a higher suppression effect.

### (4) Comparison of carbon chain length

Hexamethonium bromide has a structure where an amino group is bound by a hydrocarbon chain with 6 carbons. This was compared with decamethonium bromide having a longer hydrocarbon chain length (number of carbons: 10). Decamethonium bromide was found to have strong toxicity. Although the administration concentration and frequency were low, action to exacerbate atopic dermatitis was confirmed.

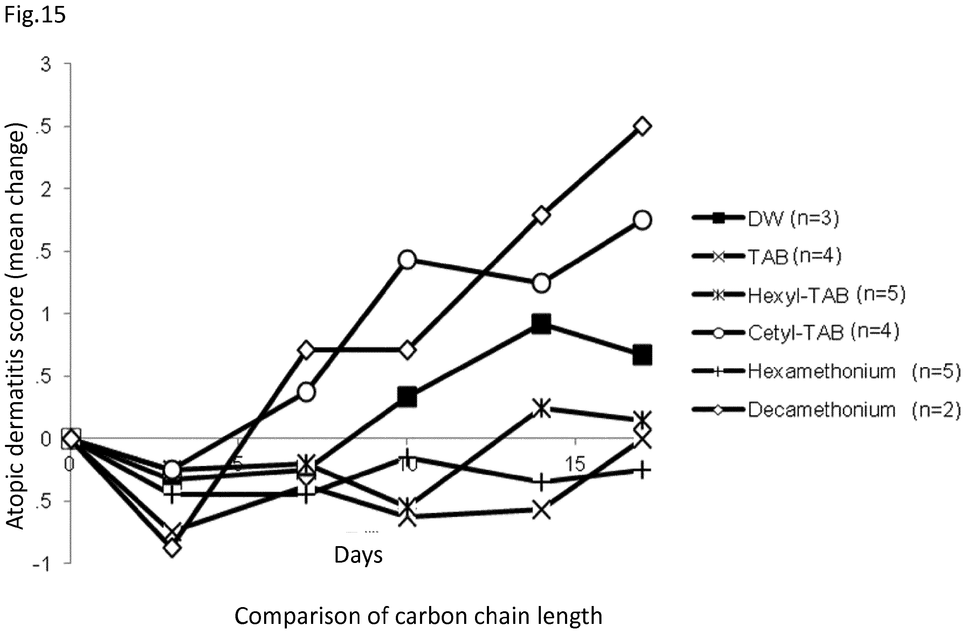

Relative to the carbon chain length of Hexyl-TAB (number of carbons: 6), TAB with a shorter chain length (number of carbons: 1) and Cetyl-TAB with a longer chain length (number of carbons: 16) were compared therewith (results shown in Figure **15**). TAB and Hexyl-TAB were found to have an effect of suppressing atopic dermatitis. However, Cetyl-TAB was confirmed to have exacerbating action. The above result shows that a tertiary amine with a hydrocarbon chain or alkyl group with less than 10 carbons has an effect of suppressing acetylcholine. It is also known that TAB and Hexyl-TAB are partial agonists of an acetylcholine receptor. Partial agonists were also demonstrated to exhibit a therapeutic effect on atopic dermatitis.

The above results suggest that a substance having one or more tertiary amino groups within the molecule and having a hydrocarbon chain with a length of less than 10 carbons is effective for suppressing atopic dermatitis.

### (Example 6: Replica method for effect of improving goose bump-like skin symptoms)

In many cases, atopic dermatitis patients have goose bump-like skin. This can be understood to be due to a high acetylcholine concentration around skin pores. This Example examined whether the degree of protrusion of skin pores in atopic dermatitis patients applied with hexamethonium decreases by using a replica method.

### (Materials and Methods)

Hexamethonium (12 mM) was applied to (back part of) the neck of atopic dermatitis patients to obtain replica images of skin before application and after 5 to 10 minutes from application. The replica images were collected by applying silicon resin (SILFLO) so as to cover the skin with outbreak of atopic dermatitis and peeling of the cured silicon from the skin after 5 minutes. Light was irradiated onto the collected replicas from one direction to compare and examine, and take pictures of, the unevenness of the skin surface.

### (Results)

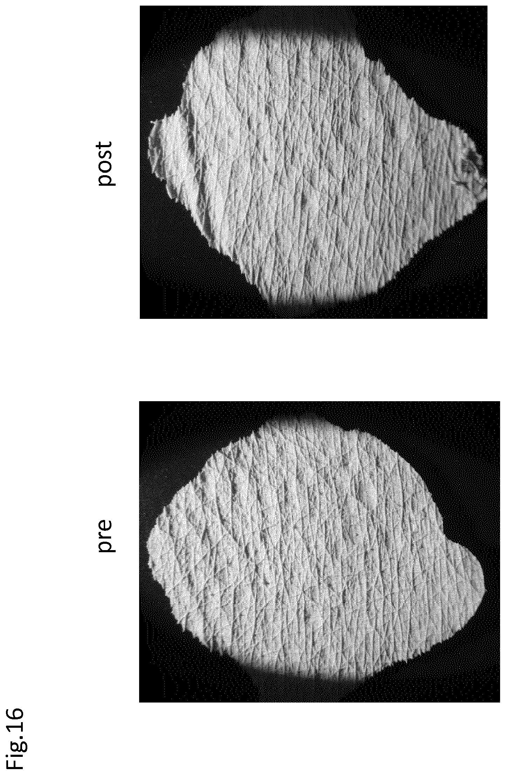

Figure **16** shows replica images before and after the application of hexamethonium. Recessed portions correspond to skin pores, and a bulge at the funneled portion of the skin pore opening indicates the protruding state of skin pores. In the replica images after application of hexamethonium, it was observed that the degree of protrusion of skin pores has subsided to be less goose bump-like. Therefore, it is understood that hexamethonium exerts anti-choline action which attenuates the action of acetylcholine, resulting in exertion of a therapeutic effect on atopic dermatitis.

### (Example 7: Combined use of nicotinic acetylcholine receptor inhibitor and muscarinic acetylcholine receptor inhibitor)

This Example confirmed the therapeutic effect from combined use of a nicotinic acetylcholine receptor inhibitor and muscarinic acetylcholine receptor inhibitor.

### (Materials and Methods)

### Preparation of atopic dermatitis model mice:

10 week old NC/Nga mice (Charles River Laboratories Japan) were used as atopic dermatitis model animals. A mite antigen (Biostir AD, Wako Pure Chemical) was used to induce dermatitis. Model mice were prepared and scored according to the method described in the package insert of Biostir AD (same as Example 1).

### Preparation of test formulation:

As an inhibitor of a muscarinic acetylcholine receptor, scopolamine hydrobromide trihydrate (hereinafter, scopolamine; Tokyo Chemical Industry) was used. As an inhibitor of a nicotinic acetylcholine receptor, hexamethonium bromide (hereinafter, hexamethonium; sigma-aldrich) was used. Each inhibitor was dissolved in purified water to prepare low concentration groups (Hexa. & Scop. Lo.) and high concentration groups (Hexa. & Scop. Hi.) with a final concentration of 6 mM and 12 mM, respectively, for each of hexamethonium and scopolamine. Purified water (DW) was used as a negative control.

### Test method:

A test formulation was applied to the back of atopic dermatitis induced mice once a day, 5 times a week, for a total of 10 times in the low concentration groups and once a day, 3 times a week, for a total of 6 times in the high concentration groups. 100 µL of a test formulation was applied to the back, and 25 µL was applied to each of the left and right auricles. Dermatitis scores were evaluated after 0, 7, and 10 days from administration. Mean change from day 0 was compared. Atopic dermatitis was induced with mite antigens only on Mondays every week to maintain the symptom.

### (Results)

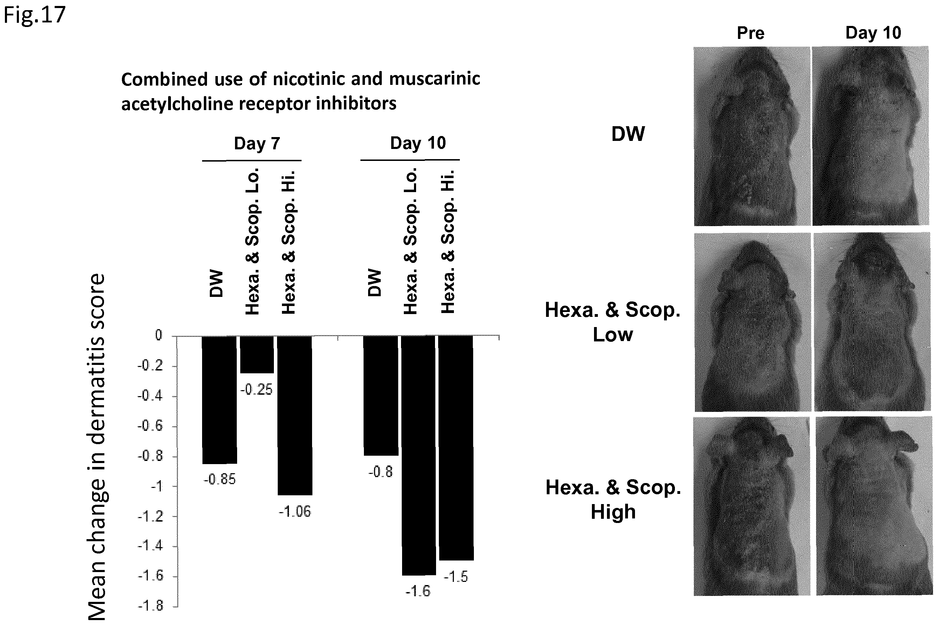

The results are shown in Figure 17. While an effect of improvement in dermatitis was not observed on day 7 of application in the low concentration groups, an effect of improvement was observed on day 10. Improvement in dermatitis was observed from day 7 of application in the high concentration groups despite fewer administration of test formulation than the low concentration groups, and a higher effect of improvement was observed on day 10 than on day 7. These results confirm that combined use of a nicotinic acetylcholine receptor inhibitor and muscarinic acetylcholine receptor inhibitor exhibits a therapeutic effect on atopic dermatitis. It was also confirmed that the effects are concentration dependent, where a therapeutic effect is manifested earlier with a higher concentration.

### (Example 8: Therapeutic effect from oral administration)

This Example studied whether a therapeutic effect is exerted from systemic administration (e.g., oral administration).

### (Materials and Methods)

### Preparation of atopic dermatitis model mice:

10 week old NC/Nga mice (Charles River Laboratories Japan) were used as atopic dermatitis model animals. A mite antigen (Biostir AD, Wako Pure Chemical) was used to induce dermatitis. Model mice were prepared and scored according to the method described in the package insert of Biostir AD (same as Example 1).

### Preparation of test formulation:

As an inhibitor of a nicotinic acetylcholine receptor, hexamethonium bromide (hereinafter, hexamethonium; sigma-aldrich) was used. The inhibitor was dissolved in purified water and prepared to have a final concentration of 60 mM. Purified water (DW) was used as a negative control.

### Test method:

150 µL of a test formulation was orally administered to atopic dermatitis induced mice once a day, 5 times a week, for a total of 10 times. Dermatitis scores were evaluated after 0 and 10 days from administration. Mean change from day 0 was compared. Atopic dermatitis was induced with mite antigens only on Mondays every week to maintain the symptom.

### (Results)

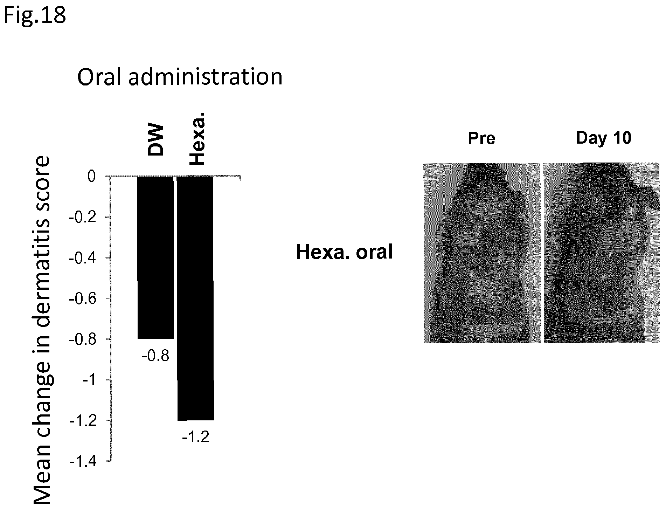

Results are shown in Figure **18****.** Improvement in the dermatitis scores was observed on day 10 of administration. The result confirms that hexamethonium has an effect of improving atopic dermatitis regardless of the route of administration.

As disclosed above, the present invention is exemplified by the use of its preferred embodiments. However, it is understood that the scope of the present invention should be interpreted based solely on the Claims. It is also understood that any patent, any patent application, and any references cited herein should be incorporated herein by reference in the same manner as the contents are specifically described herein. The present application claims priority to Japanese Patent Application No. 2016-93918 filed on May 9, 2016. The entire content thereof is incorporated herein by reference.

### [Industrial Applicability]

A composition or combination or a method for effectively treating or preventing atopic dermatitis is provided. A technology available to industries (pharmaceutical and the like) associated with formulations and the like is provided based on such a technology.

## Claims

1. A composition for treating or preventing atopic dermatitis, comprising an inhibitor of acetylcholine.

2. The composition of claim 1, wherein the inhibitor of acetylcholine is selected from the group consisting of an anti-choline agent, acetylcholinesterase, a nucleic acid encoding acetylcholinesterase, an antibody blocking the binding of acetylcholine to an acetylcholine receptor, a nucleic acid encoding an antibody blocking the binding of acetylcholine to an acetylcholine receptor, an antibody to an acetylcholine receptor, a nucleic acid encoding an antibody to an acetylcholine receptor, an agent inhibiting the gene expression of an acetylcholine receptor, and an inhibitor of choline acetyltransferase.

3. The composition of claim 1, wherein the inhibitor of acetylcholine is an anti-choline agent.

4. The composition of claim 1, which is free of crystal violet.

5. The composition of claim 3, wherein the anti-choline agent is an antagonist of an acetylcholine receptor.

6. The composition of claim 5, wherein the antagonist of an acetylcholine receptor is selected from the group consisting of trihexyphenidyl hydrochloride, scopolamine butylbromide, pirenzepine hydrochloride hydrate, ipratropium bromide, oxitropium bromide, tiotropium bromide, atropine sulfate, tropicamide, diphenhydramine hydrochloride, dicycloverine, oxybutynin hydrochloride, cyclopentrate hydrochloride, tolterodine tartrate, solifenacin succinate, darifenacin hydrobromide, mevevelin hydrochloride, procyclidine hydrochloride, acridinium bromide, propantheline bromide, scopolamine hydrobromide hydrate, metoscopolamine bromide, mebenzolate bromide, methanethelinium bromide, orphenadrine citrate, homatropine hydrobromide, prefinium bromide, methixene hydrochloride, pipethanate ethobromide, adiphenin hydrochloride, mazaticol hydrochloride hydrate, eutropin chloride, imidafenacin, fesoterodine fumarate, suxamethonium chloride hydrate, decamethonium bromide, vecuronium bromide, pancuronium bromide, d-tubocurarine chloride hydrochloride hydrate, gallamintriethiodide, mecamylamine hydrochloride, trimetaphan camsilate, hexamethonium bromide, atracurium besilate, doxacurium chloride, mivacurium chloride, 18-methoxycoronaridine, dextromethorphan hydrobromide hydrate, methyllycaconitine, α-bungarotoxin, α-conotoxin G1, benzoquinonium, and bPiDDB, and analogs, mimetics, and derivatives of these substances with anti-choline action.

7. The composition of claim 3, wherein the anti-choline agent is a partial agonist or an inverse agonist of an acetylcholine receptor.

8. The composition of claim 7, wherein the anti-choline agent is a partial agonist of an acetylcholine receptors selected from the group consisting of TAB, Hexyl-TAB, sabcomeline, and milameline, and analogs, mimetics, and derivatives of these substances with anti-choline action.

9. The composition of claim 7, wherein the anti-choline agent is an inverse agonist of an acetylcholine receptor selected from the group consisting of pirenzepine and analogs, mimetics, and derivatives of these substances with anti-choline action.

10. The composition of claim 3, wherein the anti-choline agent is a compound having a structure comprising one or more tertiary amino groups and an alkyl chain with 1 to 9 carbons binding thereto.

11. The composition of claim 10, wherein the number of carbons of the alkyl chain is 1 to 6.

12. The composition of claim 10, wherein three substituents on nitrogen of the tertiary amino group are methyl groups.

13. The composition of claim 1, wherein the inhibitor of acetylcholine is acetylcholinesterase.

14. The composition of claim 1, wherein the inhibitor of acetylcholine is an antibody blocking the binding of acetylcholine to an acetylcholine receptor.

15. The composition of claim 1, wherein the inhibitor of acetylcholine is an agent inhibiting the gene expression of an acetylcholine receptor.

16. The composition of claim 1, wherein the inhibitor of acetylcholine is an inhibitor of choline acetyltransferase.

17. A composition or combination for treating or preventing atopic dermatitis, comprising an inhibitor of muscarinic acetylcholine and an inhibitor of nicotinic acetylcholine.

18. The composition or combination of claim 17, wherein the inhibitor of muscarinic acetylcholine is an antagonist of a muscarinic acetylcholine receptor, and the inhibitor of nicotinic acetylcholine is an antagonist of a nicotinic acetylcholine receptor.

19. The composition or combination of claim 18,
wherein the antagonist of a muscarinic acetylcholine receptor is selected from the group consisting of trihexyphenidyl hydrochloride, scopolamine butylbromide, pirenzepine hydrochloride hydrate, ipratropium bromide, oxitropium bromide, tiotropium bromide, atropine sulfate, tropicamide, diphenhydramine hydrochloride, dicycloverine, oxybutynin hydrochloride, cyclopentrate hydrochloride, tolterodine tartrate, solifenacin succinate, darifenacin hydrobromide, mevevelin hydrochloride, procyclidine hydrochloride, acridinium bromide, propantheline bromide, scopolamine hydrobromide hydrate, metoscopolamine bromide, mebenzolate bromide, methanethelinium bromide, orphenadrine citrate, homatropine hydrobromide, prefinium bromide, methixene hydrochloride, pipethanate ethobromide, adiphenin hydrochloride, mazaticol hydrochloride hydrate, eutropin chloride, imidafenacin, and fesoterodine fumarate, and
wherein the antagonist of a nicotinic acetylcholine receptor is selected from the group consisting of suxamethonium chloride hydrate, decamethonium bromide, vecuronium bromide, pancuronium bromide, d-tubocurarine chloride hydrochloride hydrate, gallamintriethiodide, mecamylamine hydrochloride, trimetaphan camsilate, hexamethonium bromide, atracurium besilate, doxacurium chloride, mivacurium chloride, 18-methoxycoronaridine, dextromethorphan hydrobromide hydrate, methyllycaconitine, α-bungarotoxin, α-conotoxin G1, benzoquinonium, and bPiDDB.

20. The composition or combination of claim 19,
wherein the antagonist of a muscarinic acetylcholine receptor is selected from the group consisting of solifenacin succinate, acridinium bromide, and scopolamine hydrobromide hydrate, and
wherein the antagonist of a nicotinic acetylcholine receptor is selected from the group consisting of d-tubocurarine chloride hydrochloride hydrate, gallamintriethiodide, hexamethonium bromide, dextromethorphan hydrobromide hydrate, and α-bungarotoxin.

21. The composition or combination of claim 20, wherein the antagonist of a muscarinic acetylcholine receptor is scopolamine hydrobromide hydrate, and the antagonist of a nicotinic acetylcholine receptor is hexamethonium bromide.

22. The composition or combination of any one of claims 1 to 21, **characterized in that** the composition or combination is administered by topical application, transdermal administration, intradermal administration, subcutaneous administration, intravenous administration, oral administration, enteral administration, pulmonary administration, transnasal administration, intraperitoneal administration, ear drops, or eye drops.
